# EUROPEAN PATENT APPLICATION

(11) **EP 1 862 456 A1**
(43) Date of publication of application: **05.12.2007**
(21) Application number: 06729475.1
(22) Date of filing: 20.03.2006
(51) Int. Cl.: C07D 207/14, C07D 207/16, C07D 477/00, A61K 31/407, A61P 31/04

(54) **PROCESS FOR PRODUCING CARBAPENEM DERIVATIVE HAVING 1-ALKYLPYRROLIDINE STRUCTURE**

(30) Priority: 22.03.2005 JP 2005080840
(71) Applicant: Daiichi Sankyo Company, Limited, Tokyo 103-8426 (JP)
(72) Inventor: MICHIDA, Makoto c/o Daiichi Sankyo Company Limited, Kanagawa 254-0014 (JP); HAYASHI, Masaki c/o Daiichi Sankyo Company Limited, Kanagawa 254-0014 (JP); KOBAYASHI, Satoshi c/o Daiichi Sankyo Company Limited, Kanagawa 254-0014 (JP)
(74) Representative: Gibson, Christian John Robert
(86) International application number: PCT/JP2006/305507
(87) International publication number: WO 2006/101062

(57) **Abstract**

It is an object of the invention to find a less expensive and highly safe synthetic route for carbapenem-type antibacterial agents suitable for large-scale synthesis. A production process for the preparation of carbapenem-type antibacterial agents: 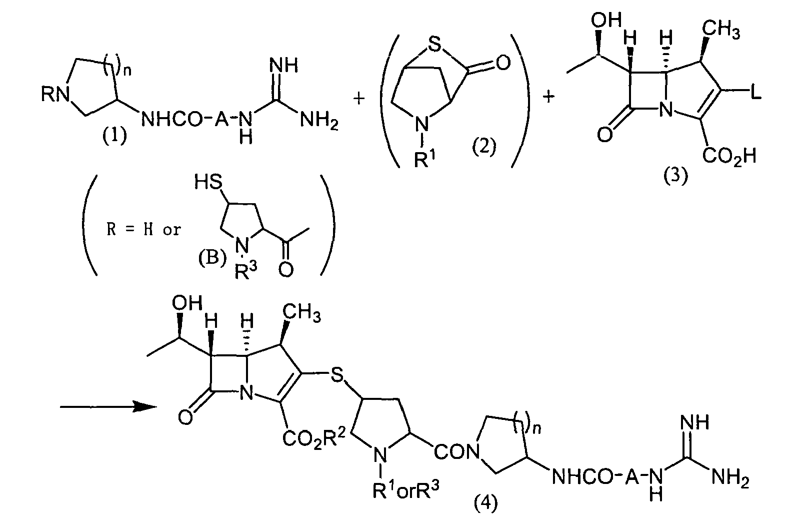 (R¹ is a C₁-C₃ alkyl group, n is 0, 1 or 2, A is a C₁-C₃ alkylene group, L is a leaving group, R³ is a hydrogen atom, a C₁-C₃ alkyl group or an amino protecting group, and the hydroxyl or carboxyl groups can independently be protected) using an amine compound (1) or a salt thereof as a synthetic intermediate is provided.

## Description

### Technical Field

The present invention relates to production processes for the preparation of 1-methylcarbapenem-type antibacterial agents having a 1-alkylpyrrolidine structure and a guanidyl group which exhibit excellent antibacterial activity, synthetic intermediates and production processes thereof.

### Background Art

Research has been conducted in recent years on the synthesis of 1-methylcarbapenem-type antibacterial agents having a 1-alkylpyrrolidine structure which exhibit excellent antibacterial activity. For example, Patent Documents 1 and 2 disclose carbapenem-type antibacterial agents having 1-alkylpyrrolidine structure at position 2 of a carbapenem skeleton and production processes thereof, as well as 1-methylcarbapenem-type antibacterial agents having a guanidyl group. However, every production process disclosed in said patent documents only contains production processes for the preparation of 1-methylcarbapenem-type antibacterial agents having a protected guanidyl group, and the patent documents do not disclose specific examples of 1-methylcarbapenem-type antibacterial agents having an unprotected guanidyl group. In addition, the production processes disclosed in said patent documents are processes for constructing three constituent partial structures in a stepwise manner, resulting in a large number of steps. Therefore, addition of protecting and deprotecting steps of a guanidyl group makes the number of steps increase further. In addition, purification by column chromatography must be frequently carried out to obtain a level of quality (purity) sufficient for use as a pharmaceutical raw material, and since the yield is not high, it is unlikely that these production processes are suitable for large-scale synthesis.

Patent Document 3 discloses a one-pot process for continuously constructing three partial structures in carbapenem-type antibacterial agents having a 1-alkylpyrrolidine structure and a guanidyl group, thereby enabling the number of steps to be reduced. In addition, since a protected carbapenem wherein the guanidyl group is protected is obtained as a solid, purification is possible to a certain extent by the simple procedure of adding water to the reaction system. However, although the resulting protected carbapenem wherein the guanidyl group is protected is obtained as a solid, due to the low purity thereof, there is the problem of requiring further purification in the subsequent deprotection step. Protected carbapenem-type antibacterial agents and precursors thereof (such as Compound (3) in the present application) are typically poorly soluble in organic solvents, and solvents having a high boiling point and high polarity are frequently used as a reaction solvent. The presence of these reaction solvents having high boiling point and high polarity impairs crystallization of the resulting protected carbapenem and hinders large-scale synthesis due to, for example, reduced product purity and making it difficult to remove solvent during the purification procedure by, for example, requiring a special concentrating apparatus for removing the solvent.

Patent Document 4 discloses a process for producing a protected carbapenem and carrying out a subsequent deprotection step while the reaction solvent is still present, without isolating the protected carbapenem. Although said patent document does not disclose any specific examples of a carbapenem-type antibacterial agent having a 1-alkylpyrrolidine structure and a guanidyl group, according to this process, purification of the target compound following deprotection is required in lieu of being able to omit isolation and purification of the protected carbapenem. In addition, since a solvent having high boiling point and high polarity used in the production step of the protected carbapenem remains in the deprotection step as well, it is necessary to use a large amount of reaction solvent in the deprotection step, thereby resulting in a considerable burden in terms of equipment and cost.

Patent Document 5 discloses a 3-substituted pyrrolidine having an unprotected guanidyl group, which can be a synthetic intermediate for the aforementioned 1-methylcarbapenem-type antibacterial agent having a 1-alkylpyrrolidine structure and a guanidyl group. However, the document provides a detailed disclosure of a production process of a 3-substituted pyrrolidine having a protected guanidyl group, a 3-substituted pyrrolidine having unprotected guanidyl group is not specifically disclosed.
[Patent Document 1] Japanese Patent Application (Kokai) No. Hei 10-204086
[Patent Document 2] Japanese Patent Application (Kokai) No. Hei 11-71277
[Patent Document 3] Japanese Patent Application (Kokai) No. 2002-212183
[Patent Document 4] Japanese Patent Application (Kokai) No. 2003-128674
[Patent Document 5] Japanese Patent Application (Kokai) No. 2001-114759

### Disclosure of the Invention

### [Problems to be Solved by the Invention]

Although carbapenem-type antibacterial agents have excellent antibacterial activity, they typically have a complex structure, thereby resulting in the need for a less expensive and highly safe synthesis route suitable for large-scale synthesis.

In addition, although a 3-substituted pyrrolidine having an unprotected guanidyl group of the present invention is a partial structure in a carbapenem-type antibacterial agent, that is a desired production compound, and is an extremely important synthetic intermediate in the aforementioned synthesis route, a process is required that enables said compound to be produced inexpensively, easily, safely and in large scale. In particular, if it becomes to be possible to acquire said synthetic intermediate stably, with high quality, at high yield and in the form of stable crystals offering greater ease of handling, it would be easy to stabilize the quality of the desired final compound obtained from the deprotection step, that is the final step, thereby making it possible consistently to produce carbapenem-type antibacterial agents having high quality.

### [Means for Solving the Problems]

As a result of conducting extensive studies on a process for producing 1-methylcarbapenem-type antibacterial agents having a 1-alkylpyrrolidine structure and a guanidyl group which exhibit excellent antibacterial activity in order to solve the aforementioned problems, the inventors of the present invention found that by using an amine or thiol compound not having a protecting group for the guanidyl group as an amino or thiol compound having a guanidyl group, that is a partial structure for producing said 1-methylcarbapenem-type antibacterial agents, even if a solvent having high boiling point and high polarity is used for the reaction in the production step for production of a protected carbapenem, a protected carbapenem or a salt thereof can be easily acquired at high yield, with high quality and in the form of stable crystals, and the solvent having high boiling point and high polarity can be easily removed, thereby leading to completion of the present invention.

The synthetic intermediate of the present invention is
(1) an amine or a thiol compound or a salt thereof represented by the formula: 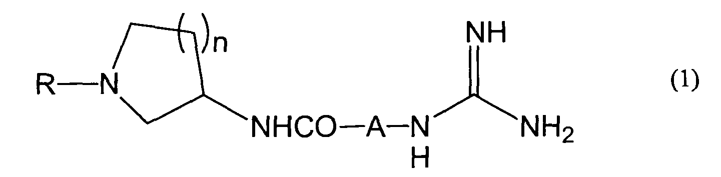 [wherein R represents a hydrogen atom or a group represented by the formula: 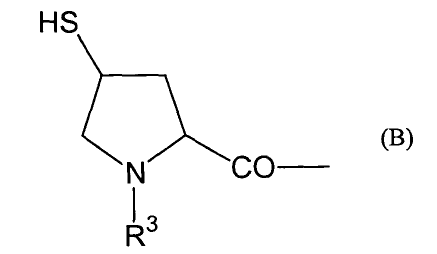 (wherein R³ represents a hydrogen atom, a C₁-C₃ alkyl group or an amino protecting group), n represents 0, 1 or 2 and A represents a C₁-C₃ alkylene group], preferably,
(2) a compound or a salt thereof, wherein R³ is a C₁-C₃ alkyl group,
(3) a compound or a salt thereof, wherein R is a hydrogen atom,
(4) a compound or a salt thereof, wherein n is 0 or 1,
(5) a compound or a salt thereof, wherein n is 1, or
(6) a compound or a salt thereof, wherein A is a methylene group.
   The production process of the present invention wherein R is a hydrogen atom is,
(7) a process for the production of a compound or a salt thereof represented by the formula: 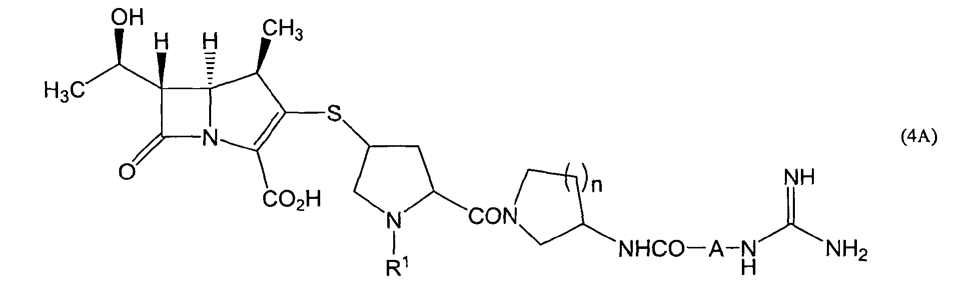 (wherein R¹ represents a C₁-C₃ alkyl group, and n and A have the same meanings as defined above respectively) comprising a step for preparing a compound or a salt thereof represented by the above formula (4A) (wherein n, A and R¹ have the same meanings as defined above respectedly and the hydroxyl or carboxyl groups can independently be protected) by reacting a compound or a salt thereof represented by the formula: 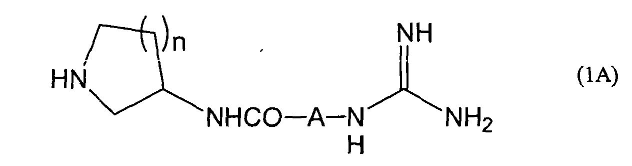 (wherein n and A have the same meanings as defined above respectively), a compound or a salt thereof represented by the formula: 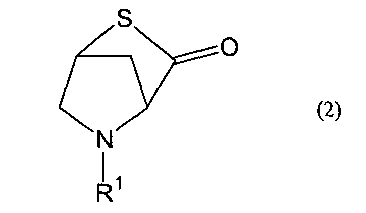 (wherein R¹ has the same meaning as defined above), and a compound or a salt thereof represented by the formula: 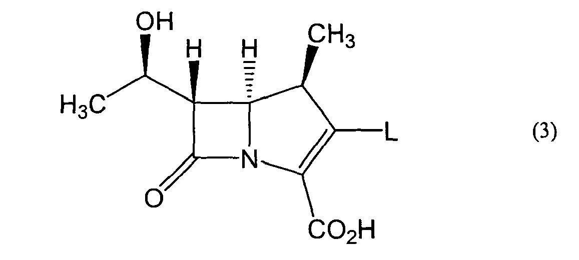 (wherein L represents a leaving group, and the hydroxyl or carboxyl groups can independently be protected) in the presence of a base in an inert solvent, preferably,
(8) a production process, wherein R¹ is a methyl group,
(9) a production process, wherein n is 0 or 1,
(10) a production process, wherein n is 1,
(11) a production process, wherein A is a methylene group,
(12) a production process, wherein L is a diarylphosphoryloxy group,
(13) a production process, wherein L is a diphenylphosphoryloxy group,
(14) a production process, wherein R2 is a carboxyl protecting group,
(15) a production process, wherein compound (4A) or the salt thereof is isolated and purified by crystallization, or
(16) a production process, wherein the inert solvent in the step for preparing compound (4A) or the salt thereof is dimethylformamide, dimethylacetamide, dimethyl sulfoxide or hydrous dimethyl sulfoxide.
   A production process of the present invention wherein R is a group represented by formula (B) is
(17) a process for the production of a compound or a salt thereof represented by the formula: 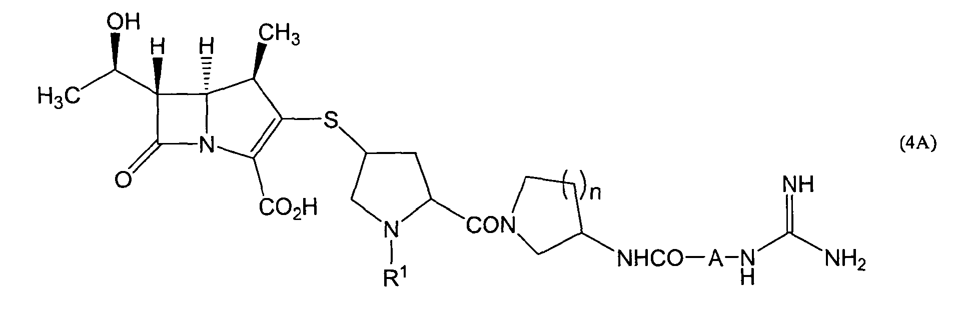 (wherein n, A and R¹ have the same meanings as defined above respectively) comprising a step for preparing a compound or a salt thereof represented by the formula: 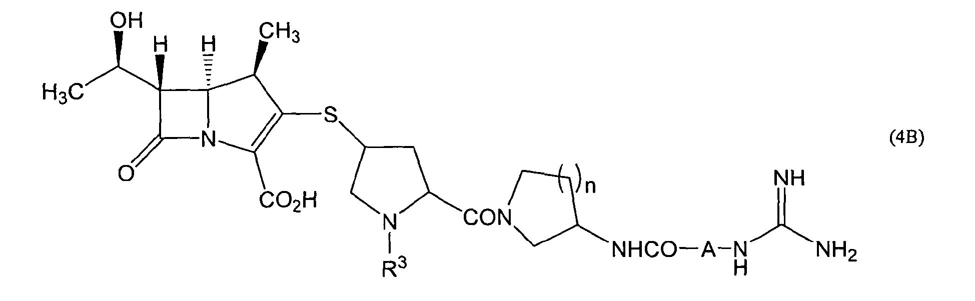 (wherein n, A and R³ have the same meanings as defined above respectively, and the hydroxyl or carboxyl groups can independently be protected) by reacting a compound or a salt thereof represented by the formula: 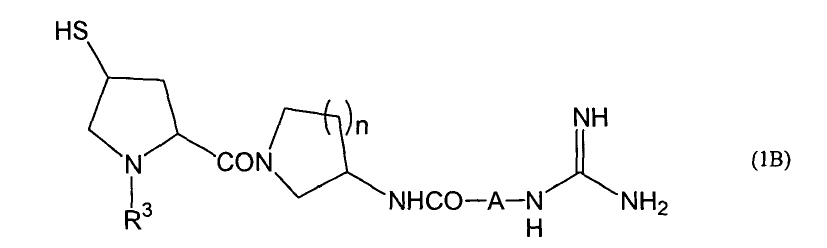 (wherein n, A and R³ have the same meanings as defined above respectively), and a compound or a salt thereof represented by the formula: 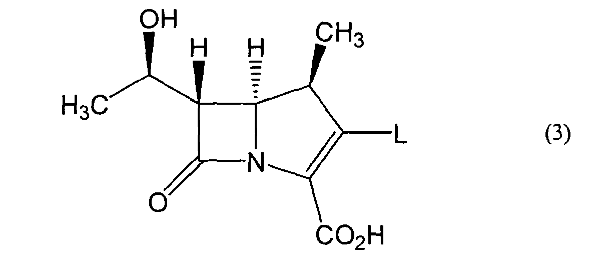 (wherein L has the same meaning as defined above respectively, and the hydroxyl or carboxyl groups can independently be protected) in the presence of a base in an inert solvent, preferably,
(18) a production process, wherein R¹ and R³ are each a methyl group,
(19) a production process, wherein n is 0 or 1,
(20) a production process, wherein n is 1,
(21) a production process, wherein A is a methylene group,
(22) a production process, wherein L is a diarylphosphoryloxy group,
(23) a production process, wherein L is a diphenylphosphoryloxy group,
(24) a production process, wherein R² is a carboxyl protecting group, or
(25) a production process, wherein compound (4B) or a salt thereof is isolated and purified by crystallization.
   The production process of compound (1A) of the present invention is
(26) a process for the production of a compound (1A) or a salt thereof represented by the formula: 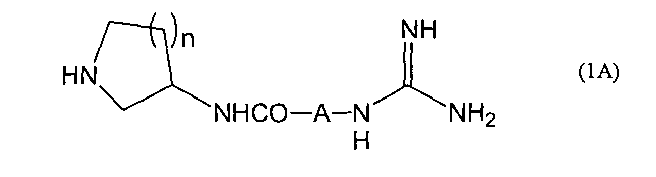 (wherein n and A have the same meanings as defined above respectively) by reacting a compound or a salt thereof represented by the formula: 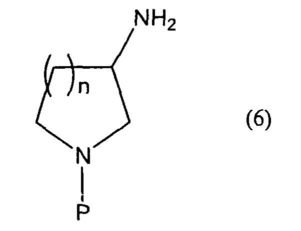 (wherein n has the same meaning as defined above and P represents an amino protecting group) with a compound represented by the formula: 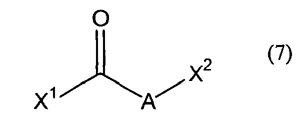 (wherein A has the same meaning as defined above and X¹ and X² each independently represent a leaving group) to prepare a compound or a salt thereof represented by the formula: 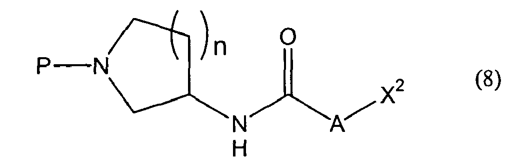 (wherein n, A, P and X² have the same meanings as defined above respectively), by then reacting compound (8) or a salt thereof with a reagent providing a source of ammonia to prepare a compound or a salt thereof represented by the formula: 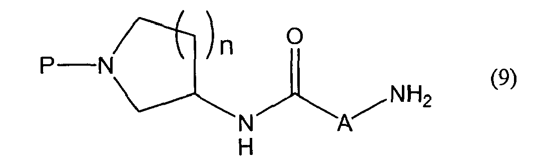 (wherein n, A and P have the same meanings as defined above respectively), by then reacting compound (9) or a salt thereof with a compound or a salt thereof represented by the formula: 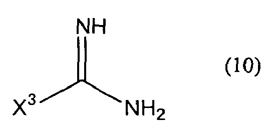 (wherein X³ represents a leaving group) to prepare a compound or a salt thereof represented by the formula: 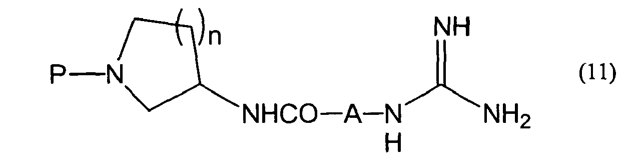 (wherein n, A and P have the same meanings as defined above respectively), and by finally removing the group P from compound (11) or a salt thereof by a deprotection reaction.

In R¹ and R³ of the present invention, the "C₁-C₃ alkyl group" is a straight or branched alkyl group having from 1 to 3 carbon atoms and can be, for example, a methyl, ethyl, propyl or isopropyl group, preferably a C₁-C₂ alkyl group, more preferably a methyl group.

In A of the present invention, the "C₁-C₃ alkylene group" is a straight or branched alkylene group having from 1 to 3 carbon atoms and can be, for example, a methylene, ethylene, propylene, trimethylene or 1,1-ethylene group, preferably a methylene group.

In L of the present invention, the "leaving group" is not particularly limited so long as it is a group eliminated as a usual nucleophilic residue, for example, as described in Japanese Patent Application (Kokai) No. Hei-11-71277 and can be, for example, a halogen atom such as chlorine, bromine or iodine; a tri-halogeno-methyloxy group such as trichloromethyloxy; a lower alkanesulfonyloxy group such as methanesulfonyloxy or ethanesulfonyloxy; a halogeno-lower alkanesulfonyloxy group such as trifluoromethanesulfonyloxy or pentafluoroethanesulfonyloxy; an arylsulfonyloxy group such as benzenesulfonyloxy, p-toluenesulfonyloxy or p-nitrobenzenesulfonyloxy; or a diarylphosphoryloxy group such as diphenylphosphoryloxy, preferably a diarylphosphoryloxy group, more preferably a diphenylphosphoryloxy group (-O-P(=O)(OPh)₂).

In the present invention, the "hydroxyl protecting group" can be, for example, "aliphatic acyl groups" including an alkylcarbonyl group such as a formyl, acetyl, propionyl, butyryl, isobutyryl, pentanoyl, pyvaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, eicosanoyl or heneicosanoyl group; a carboxylated alkylcarbonyl group such as a succinoyl, glutaroyl or adipoyl group; a halogeno-lower alkylcarbonyl group such as a chloroacetyl, dichloroacetyl, trichloroacetyl or trifluoroacetyl group; a lower alkoxy-lower alkylcarbonyl group such as a methoxyacetyl group; and an unsaturated alkylcarbonyl group such as a (E)-2-methyl-2-butenoyl group; "aromatic acyl groups" including an arylcarbonyl group such as a benzoyl, α-naphthoyl or β-naphthoyl group; a halogenoarylcarbonyl group such as a 2-bromobenzoyl or 4-chlorobenzoyl group; a lower alkylated arylcarbonyl group such as a 2,4,6-trimethylbenzoyl or 4-toloyl group; a lower alkoxylated arylcarbonyl group such as an 4-anisoyl group; a carboxylated arylcarbonyl group such as a 2-carboxybenzoyl, 3-carboxybenzoyl or 4-carboxybenzoyl group; a nitrated arylcarbonyl group such as a 4-nitrobenzoyl or 2-nitrobenzoyl group; a lower alkoxycarbonylated arylcarbonyl group such as a 2-(methoxycarbonyl)benzoyl group; and an arylated arylcarbonyl group such as a 4-phenylbenzoyl group; "tetrahydropyranyl or tetrahydrothiopyranyl groups" such as a tetrahydropyran-4-yl, 3-bromotetrahydropyran-2-yl, 4-methoxytetrahydropyran-4-yl, tetrahydrothiopyran-2-yl or 4-methoxytetrahydrothiopyran-4-yl group; "tetrahydrofuranyl or tetrahydrothiofuranyl groups" such as a tetrahydrofuran-2-yl or tetrahydrothiofuran-2-yl group; "silyl groups" including a tri-lower alkylsilyl group such as a trimethylsilyl, triethylsilyl, isopropyldimethylsilyl, t-butyldimethylsilyl, methyldiisopropylsilyl, methyldi-t-butylsilyl or triisopropylsilyl group; and a tri-lower alkylsilyl group substituted with 1 or 2 aryl groups such as a diphenylmethylsilyl, diphenylbutylsilyl, diphenylisopropylsilyl or phenyldiisopropylsilyl group; "alkoxymethyl groups" including a lower alkoxymethyl group such as a methoxymethyl, 1,1-dimethyl-1-methoxymethyl, ethoxymethyl, propoxymethyl, isopropoxymethyl, butoxymethyl or t-butoxymethyl group; a lower alkoxylated lower alkoxymethyl group such as a 2-methoxyethoxymethyl group; and a halogeno-lower alkoxymethyl such as a 2,2,2-trichloroethoxymethyl or bis(2-chloroethoxy)methyl group; "substituted ethyl groups" including a lower alkoxylated ethyl group such as a 1-ethoxyethyl or 1-(isopropoxy)ethyl group; and a halogenated ethyl group such as a 2,2,2-trichloroethyl group; "aralkyl groups" including a lower alkyl group substituted with 1 to 3 aryl groups such as a benzyl, α-naphthylmethyl, β-naphthylmethyl, diphenylmethyl, triphenylmethyl, α-naphthyldiphenylmethyl or 9-anthrylmethyl group; and a lower alkyl group substituted with 1 to 3 aryl groups in which the aryl ring is substituted with lower alkyl, lower alkoxy, halogen or cyano group(s) such as a 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl, 4-cyanobenzyl, methyl or piperonyl group; "alkoxycarbonyl groups" including a lower alkoxycarbonyl group such as a methoxycarbonyl, ethoxycarbonyl, t-butoxycarbonyl or isobutoxycarbonyl group; and a lower alkoxycarbonyl group substituted with halogen or tri-lower alkylsilyl group(s) such as a 2,2,2-trichloroethoxycarbonyl or 2-trimethylsilylethoxycarbonyl group; "alkenyloxycarbonyl groups" such as a vinyloxycarbonyl or allyloxycarbonyl group; and "aralkyloxycarbonyl groups" in which the aryl ring may be substituted with 1 or 2 lower alkoxy or nitro groups such as a benzyloxycarbonyl, 4-methoxybenzyloxycarbonyl, 3,4-dimethoxybenzyloxycarbonyl, 2-nitrobenzyloxycarbonyl or 4-nitrobenzyloxycarbonyl group, preferably tetrahydropyranyl or tetrahydrothiopyranyl groups, tetrahydrofuranyl or tetrahydrothiofuranyl groups, silyl groups, alkoxymethyl groups, aralkyl groups or aralkyloxycarbonyl groups.

In the present invention, the "carboxyl protecting group" can be, for example, a benzyl group which may be substituted such as a benzyl, p-nitrobenzyl, o-nitrobenzyl, p-methoxybenzyl, 2,4-dimethoxybenzyl or trimethylbenzyl group; or an allyl group which may be substituted at the 2-position such as an allyl, 2-chloroallyl or 2-methylallyl group, preferably a benzyl group which may be substituted, more preferably a p-nitrobenzyl group.

n of the present invention is preferably 0 or 1, more preferably 1.

R of the present invention is preferably a hydrogen atom.

The "amino protecting group" in R³ and P of the present invention can be, for example, a benzyl group which may be substituted at an aromatic ring such as a benzyl, p-nitrobenzyl, o-nitrobenzyl, p-methoxybenzyl, 2,4-dimethoxybenzyl or trimethylbenzyl group; an allyl group which may be substituted at the 2-position such as an allyl, 2-chloroallyl or 2-methylallyl group; a benzyloxycarbonyl group which may be substituted at an aromatic ring such as a benzyloxycarbonyl, 4-nitrobenzyloxycarbonyl, 4-methoxybenzylcarbonyl, 4-chlorobenzylcarbonyl or 4-methylbenzylcarbonyl group; an alkoxycarbonyl group which may be substituted such as a methoxycarbonyl, ethoxycarbonyl, t-butoxycarbonyl or 2,2,2-trichloroethoxycarbonyl group; or an alkenyloxycarbonyl group which may be substituted such as an allyloxycarbonyl, 2-chloroallyloxycarbonyl or vinyloxycarbonyl group, preferably a benzyl, 4-nitrobenzyloxycarbonyl, t-butoxycarbonyl or allyloxycarbonyl group, more preferably in P, a benzyl or t-butoxycarbonyl group, more preferably in R³, a 4-nitrobenzyloxycarbonyl group.

The "leaving group" in X¹ of the present invention can be, for example, a halogen atom such as chlorine or bromine, a trimethylsilyloxy group, a methanesulfonyloxy group or an acid anhydride, preferably a chlorine or bromine atom.

The "leaving group" in X² of the present invention can be, for example, a halogen atom such as chlorine, bromine or iodine or an activated hydroxyl group such as a methanesulfonyloxy group, preferably a halogen atom, more preferably a chlorine atom.

The "leaving group" in X³ of the present invention can be an alkoxy group such as methoxy, ethoxy or benzyloxy, an alkylthio group such as methylthio, ethylthio or benzylthio, an imidazolyl group, a pyrazolyl group or a triazolyl group, preferably a methylthio, pyrazolyl or triazolyl group, more preferably a pyrazolyl group.

Compound (4A) or (4B) of the present invention can form a salt with an acidic compound. The acidic compound can be, for example, an inorganic acid such as hydrofluoric acid, hydrochloric acid, hydrobromic acid, hydroiodic acid, sulfuric acid, nitric acid, phosphoric acid or carbonic acid; an organic carboxylic acid such as formic acid, acetic acid, trifluoroacetic acid, oxalic acid or phthalic acid; or an organic sulfonic acid such as methanesulfonic acid, trifluoromethanesulfonic acid, benzenesulfonic acid or p-toluenesulfonic acid, preferably an inorganic acid, more preferably hydrochloric acid or sulfuric acid.

Compound (1) of the present invention can form a salt with an acidic compound. The acidic compound can be, for example, an inorganic acid such as hydrochloric acid, sulfuric acid, nitric acid or phosphoric acid; an organic carboxylic acid such as formic acid, acetic acid, trifluoroacetic acid, oxalic acid or phthalic acid; or an organic sulfonic acid such as methanesulfonic acid, benzenesulfonic acid, p-toluenesulfonic acid or trifluoromethanesulfonic acid, preferably an inorganic acid, more preferably hydrochloric acid or sulfuric acid.

Compound (11) of the present invention can form a salt with an acidic compound. The acidic compound can be, for example, an inorganic acid such as hydrochloric acid, sulfuric acid, nitric acid or phosphoric acid; an organic carboxylic acid such as formic acid, acetic acid, trifluoroacetic acid, oxalic acid or phthalic acid; or an organic sulfonic acid such as methanesulfonic acid, benzenesulfonic acid, p-toluenesulfonic acid or trifluoromethanesulfonic acid, preferably an inorganic acid, more preferably hydrochloric acid or sulfuric acid.

### [Effect of the Invention]

According to the present invention, a production process for the carbapenem-type antibiotics that is efficient and suitable for large-scale synthesis can be provided, which solves the following five defects in the conventional methods, i.e., 1) column chromatography is employed, 2) although it is required that a protected carbapenem is collected once, crystallization of the synthetic intermediate is difficult due to the high solubility in a solvent having high boiling point and high polarity, 3) an extraction step is required when coloring insolubles derived from the protecting group are removed in the deprotection step, 4) the whole volume becomes large in cases where operations for crystallization of the final desired compound are continuously carried out without removing the high boiling point solvent, and 5) a purification operation is required again in order to stabilize the quality after the crude crystals are isolated once.

### [Best Mode for Carrying Out the Invention]

In the present invention, the 1-methylcarbapenem-type antibacterial agents having a 1-alkylpyrrolidine structure and a guanidyl group can be prepared by the following process A or B.

### (Process A)

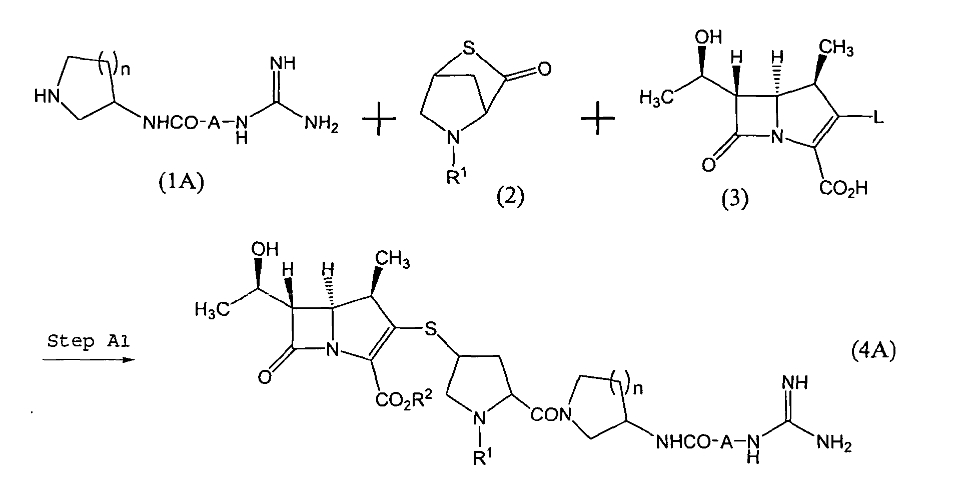

In the above formulae, n, A, R¹ and L have the same meanings as defined above. The hydroxyl or carboxyl group can independently be protected, if necessary.

### (Step A1)

The step A1 is to prepare compound (4A) or a salt thereof and is accomplished by obtaining a high purity crystal of compound (4A) or a salt thereof by reacting compound (1A) or a salt thereof, compound (2) or a salt thereof and compound (3) or a salt thereof in the presence of a base in an inert solvent.

The base employed in the present step can be, for example, an organic base such as triethylamine, diisopropylethylamine, 4-methylmorpholine, 4-ethylmorpholine, pyridine, lutidine, 4-dimethylaminopyridine, 1-methylimidazole, 1,2-dimethylimidazole, 1,8-diazabicyclo[5.4.0]-7-undecene (DBU), 1,5-diazabicyclo[4.3.0]non-5-ene (DBN) or 1,4-diazabicyclo[2.2.2]octane (TED); or inorganic bases such as lithium hydroxide, sodium hydroxide, potassium hydroxide, lithium carbonate, sodium carbonate, potassium carbonate, cesium carbonate, sodium hydrogencarbonate or potassium hydrogencarbonate, preferably triethylamine, diisopropylethylamine, sodium carbonate, potassium carbonate or sodium hydrogencarbonate.

The reaction solvent employed in the present step is not particularly limited so long as it does not inhibit the reaction and dissolves the material to some extent and can be, for example, a nitrile such as acetonitrile, an alcohol such as methanol, ethanol, propanol, isopropyl alcohol or butanol; an ester such as methyl acetate, ethyl acetate or isopropyl acetate; a halogenated hydrocarbon such as dichloromethane, dichloroethane or chloroform; an ether such as ether, dimethoxyethane, tetrahydrofuran or dioxane; a ketone such as acetone or methyl ethyl ketone; an aromatic hydrocarbon such as benzene, toluene or xylene; an amide such as dimethylformamide or dimethylacetamide; a sulfoxide such as dimethyl sulfoxide; water; or a combination of the above solvents at an arbitrary ratio, preferably a nitrile, an amide, a sulfoxide, a hydrous amide or a hydrous sulfoxide, more preferably as amide, a sulfoxide, a hydrous amide or a hydrous sulfoxide, still more preferably dimethylformamide, dimethylacetamide, dimethyl sulfoxide or hydrous dimethyl sulfoxide.

The reaction temperature of the present step varies depending mainly on the reaction solvent and is usually from -50°C to 100°C, preferably from 10°C to 50°C. The reaction time of the present step varies depending on the reaction solvent and the reaction temperature and is usually from 1 hour to 60 hours, preferably from 4 hours to 30 hours.

After completion of the reaction of the present step, the crystalline compound (4A) is produced from the reaction mixture by adding a solvent to the reaction mixture. The crystals are filtered to give high purity crystalline compound (4A).

The solvent added to the reaction mixture and employed for crystallization is not particularly limited so long as it does not affect the decomposition of compound (4A) and is a solvent having a low solubility to some extent and can be, for example, a nitrile such as acetonitrile; an alcohol such as methanol, ethanol, propanol, isopropyl alcohol, butanol or t-butyl alcohol; an ester such as methyl acetate, ethyl acetate or isopropyl acetate; a halogenated hydrocarbon such as dichloromethane, dichloroethane or chloroform; an ether such as ether, dimethoxyethane, tetrahydrofuran or dioxane; a ketone such as acetone or methyl ethyl ketone; an aromatic hydrocarbon such as benzene, toluene or xylene; water; an aqueous solution of an inorganic salt such as potassium chloride, sodium chloride, magnesium chloride or calcium chloride; or a combination of the above solvents at an arbitrary ratio, preferably an alcohol, an ester, water, an alcohol-ester mix, a water-alcohol mix, a water-ester mix, a water-ketone mix, a brine-alcohol mix, a brine-ester mix or a brine-ketone mix, more preferably an alcohol-ester mix, a brine-alcohol mix, a brine-ester mix or a brine-ketone mix, still more preferably an alcohol-ester mix or a brine-ketone mix.

Compound (4A) obtained by the present step can afford the crystalline solid as a solid salt, and the acids which form the salt can be inorganic acids such as hydrofluoric acid, hydrochloric acid, hydrobromic acid, hydroiodic acid, sulfuric acid, nitric acid, phosphoric acid or carbonic acid; organic carboxylic acids such as formic acid, acetic acid, trifluoroacetic acid, oxalic acid or phthalic acid; or organic sulfonic acids such as methanesulfonic acid, trifluoromethanesulfonic acid, benzenesulfonic acid or p-toluenesulfonic acid, preferably inorganic acids, more preferably hydrochloric acid or sulfuric acid.

Compound (1A) or a salt thereof, i.e., the starting material compound of the present step can be obtained by Process C described later.

Compound (2) or a salt thereof, i.e., the starting material compound of the present step can be obtained by Japanese Patent Application (Kokai) No. 2002-212183.

As compound (3) or a salt thereof, i.e., the starting material compound of the present step, is a commercially available compound or it can be obtained, for example, via a 2-oxo-carbapenam compound according to the description of Japanese Patent Application (Kokai) No. Hei 4-330085. Further, it is also included in the present invention that compound (3) is generated from the 2-oxo-carbapenam compound and is used for the present step without isolating compound (3).

### (Deprotecting Step)

In cases where the hydroxyl and carboxyl groups of compoud (3) are protected, after the completion of the reaction, compound (4A) can be obtained by removing the protecting group using a known method in this technical field. For example, in cases where the hydroxyl protecting group of compound (3) is a silyl group, the protecting group can be removed by treatment with a compound which can afford a fluorine anion such as tetrabutylammonium fluoride, hydrogen fluoride, hydrogen fluoride-pyridine and potassium fluoride, or treatment with an organic acid such as acetic acid, methanesulfonic acid, p-toluenesulfonic acid, trifluoroacetic acid or trifluoromethanesulfonic acid; or an inorganic acid such as hydrochloric acid. In cases where the hydroxyl protecting group of compound (3) is an aralkyl or aralkyloxycarbonyl group, the protecting group can be removed by contacting the compound with a reducing agent in a solvent (preferably catalytic hydrogenation reaction under room temperature) or using an oxdizing agent. In cases where the hydroxyl protecting group of compound (3) is an aliphatic acyl, aromatic acyl or alkoxycarbonyl group, the protecting group can be removed by treatment with a base in a solvent. In cases where the hydroxyl protecting group of compound (3) is an alkoxymethyl, tetrahydropyranyl, tetrahydrothiopyranyl, tetrahydrofuranyl, tetrahydrothiofuranyl or substituted ethyl group, the protecting group can be removed by treatment with acid in a solvent. In cases where the hydroxy protecting group of compound (3) is an alkenyloxycarbonyl group, the protecting group can be removed by treatment with a base in the same manner as the aforesaid cases where the hydroxyl protecting group is an aliphatic acyl, aromatic acyl or alkoxycarbonyl group.

In cases where the carboxyl protecting group of compound (3) is a benzyl group which may be substituted, the protecting group can be removed by reacting with hydrogen gas in water, methanol, ethanol, tetrahydrofuran or a mixed solvent thereof, in the presence of palladium-carbon or a platinum catalyst. The deprotecting agent employed in the present step can be, for example, a Zn catalyst, a Pd catalyst-hydrogen gas, a Pt catalyst-hydrogen gas or a Ni catalyst-hydrogen gas, preferably a Pd catalyst-hydrogen gas, more preferably Pd-C and hydrogen gas, Pd(OH)₂-C and hydrogen gas or Pd-zeolite and hydrogen gas. The reaction solvent employed in the present step is not particularly limited so long as it does not inhibit the present reaction and can be, for example, an ether such as tetrahydrofuran, dioxane or ether; an alcohol such as methanol, ethanol, propanol or isopropyl alcohol; an ester such as methyl acetate, ethyl acetate or isopropyl acetate; water; or a mixed solvent of these, preferably water. In cases where water is employed as the reaction solvent, it is effective that the pH of the reaction mixture is adjusted to approximately 7 for suppression of decomposition of the deprotected form, i.e., the desired product and simultaneous removal of the insoluble by-product derived from the protecting group by filtration of the catalyst after the reaction. The base added to the reaction mixture for that purpose is not particularly limited so long as it is usually used for a reaction as a base and can be, for example, a carbonate such as sodium hydrogencarbonate, sodium carbonate or potassium hydrogencarbonate, preferably sodium hydrogencarbonate. The reaction temperature of the present step is usually from 0°C to 50°C, preferably from 10°C to 40°C. The reaction time of the present step varies depending on the reaction temperature, the reaction solvent and the kind of the deprotecting agent and is usually from 5 minutes to 12 hours, preferably from 30 minutes to 6 hours.

In addition, in cases where the carboxyl protecting group of compound (3) is an allyl group which may be substituted at the 2-position, the protecting group can be removed by reacting with a trialkyltin hydride such as tributyltin hydride or an alkali metal organic carbonate such as sodium 2-ethylhexanoate.

After completion of the reaction of the present step, the desired compound (4A) can be obtained as a crystalline solid of high purity by adding a solvent to the reaction mixture after the catalyst is removed by filtration from the reaction mixture. In particular, in cases where water is employed as the reaction solvent of the present step, after completion of the reaction of the present step, the desired compound (4A) with a high yield and a high quality can be obtained by directly crystallizing it from the reaction mixture from which the catalyst was filtrated without concentrating water and carrying out a troublesome operation such as a troublesome liquid separation extraction. Further, in cases where only water is employed as the reaction solvent, the by-product derived from the protecting group precipitates in the reaction system and can be also removed simultaneously with the filtration of the catalyst, and it can be expected that the desired compound (4A) of high purity can be efficiently obtained at a high yield only by an extremely easy operation.

The solvent added to the reaction mixture and employed for the crystallization is not particularly limited so long as it does not affect decomposition of compound (4A) and is a solvent in which the compound has a low solubility to some extent, and can be, for example, an alcohol such as methanol, ethanol or isopropyl alcohol; an ether such as tetrahydrofuran or dioxane; a ketone such as acetone; a nitrile such as acetonitrile; or a mixed solvent of these with water, preferably hydrous ethanol, hydrous tetrahydrofuran or hydrous acetone.

Since compound (4A) obtained by the present step has a basic functional group, it can be obtained as a salt. The acid which forms the salt can be, for example, an inorganic acid such as hydrochloric acid, hydrobromic acid, sulfuric acid, phosphoric acid or carbonic acid; an organic carboxylic acid such as formic acid, acetic acid, oxalic acid or phthalic acid; or an organic sulfonic acid such as methanesulfonic acid or p-toluenesulfonic acid.

### (Process B)

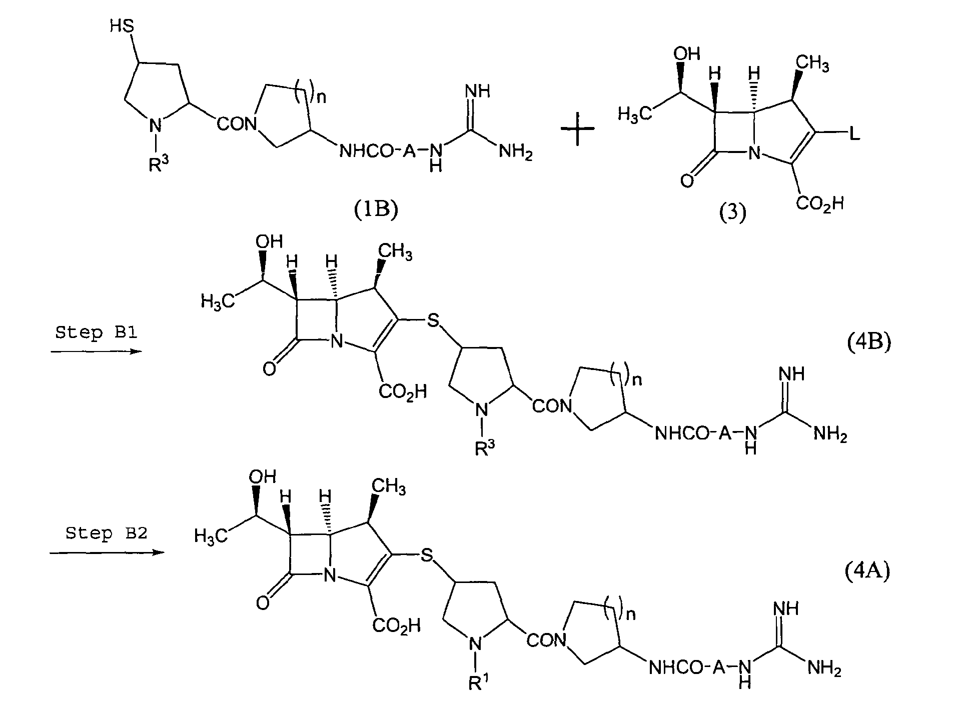

In the above formulae, n, A, R¹, R³ and L have the same meanings as defined above. The hydroxyl or carboxyl group can independently be protected, if necessary.

### (Step B1)

The step B1 is to prepare compound (4B) or a salt thereof and is accomplished by obtaining compound (4B) or a salt thereof by reacting compound (1B) or a salt thereof and compound (3) or a salt thereof in the presence of a base in an inert solvent.

The base employed in the present step can be, for example, an organic base such as triethylamine, diisopropylethylamine, 4-methylmorpholine, 4-ethylmorpholine, pyridine, lutidine, 4-dimethylaminopyridine, 1-methylimidazole, 1,2-dimethylimidazole, 1,8-diazabicyclo[5.4.0]-7-undecene (DBU), 1,5-diazabicyclo[4.3.0]non-5-ene (DBN) or 1,4-diazabicyclo[2.2.2]octane (TED); or an inorganic base such as lithium hydroxide, sodium hydroxide, potassium hydroxide, lithium carbonate, sodium carbonate, potassium carbonate, cesium carbonate, sodium hydrogencarbonate or potassium hydrogencarbonate, preferably triethylamine, diisopropylethylamine, sodium carbonate, potassium carbonate or sodium hydrogencarbonate, more preferably sodium hydrogencarbonate.

The reaction solvent employed in the present step is not particularly limited so long as it does not inhibit the reaction and dissolves the starting material to some extent, and can be, for example, a nitrile such as acetonitrile; an ester such as methyl acetate, ethyl acetate or isopropyl acetate; a halogenated hydrocarbon such as dichloromethane, dichloroethane or chloroform; an ether such as ether, dimethoxyethane, tetrahydrofuran or dioxane; an aromatic hydrocarbon such as benzene, toluene or xylene; an amide such as dimethylformamide or dimethylacetamide; a sulfoxide such as dimethyl sulfoxide; water; or a combination of the above solvents at an arbitrary ratio, preferably a nitrile, an amide, a sulfoxide or an ether, more preferably acetonitrile, dimethylformamide, dimethyl sulfoxide or tetrahydrofuran, still more preferably dimethyl sulfoxide.

The reaction temperature of the present step varies depending mainly on the reaction solvent and is usually from -20°C to 40°C, preferably from -10°C to 20°C. The reaction time of the present step varies depending on the reaction solvent and the reaction temperature and is usually from 30 minutes to 108 hours, preferably from 1 hour to 18 hours.

After completion of the reaction of the present step, a crystalline compound (4B) is produced from the reaction mixture by adding a solvent to the reaction mixture. The resulting crystalline compound is filtrated to give crystalline compound (4B) of high purity.

The solvent added to the reaction mixture and employed for the crystallization is not particularly limited so long as it does not affect decomposition of compound (4B) and is a solvent in which the compound has a low solubility to some extent, and can be, for example, a nitrile such as acetonitrile; an alcohol such as methanol, ethanol, propanol, isopropyl alcohol, butanol or t-butyl alcohol; an ester such as methyl acetate, ethyl acetate or isopropyl acetate; a halogenated hydrocarbon such as dichloromethane, dichloroethane or chloroform; an ether such as ether, dimethoxyethane, tetrahydrofuran or dioxane; a ketone such as acetone or methyl ethyl ketone; an aromatic hydrocarbon such as benzene, toluene and xylene; water; an aqueous solution of an inorganic salt such as potassium chloride, sodium chloride, magnesium chloride or calcium chloride; or a combination of the above solvents at an arbitrary ratio, preferably an alcohol, an ester, water, an alcohol-ester mix, a water-alcohol mix, a water-ester mix, a water-ketone mix, a brine-alcohol mix, a brine-ester mix or a brine-ketone mix, more preferably an alcohol-ester mix, a brine-alcohol mix, a brine-ester mix or a brine-ketone mix, still more preferably an alcohol-ester mix or a brine-ketone mix.

Compound (4B) obtained by the present step can afford a crystalline solid as a salt and the acid which forms the salt can be, for example, an inorganic acid such as hydrofluoric acid, hydrochloric acid, hydrobromic acid, hydroiodic acid, sulfuric acid, nitric acid, phosphoric acid or carbonic acid; an organic carboxylic acid such as formic acid, acetic acid, trifluoroacetic acid, oxalic acid or phthalic acid; or an organic sulfonic acid such as methanesulfonic acid, trifluoromethanesulfonic acid, benzenesulfonic acid or p-toluenesulfonic acid, preferably an inorganic acid, more preferably hydrochloric acid or sulfuric acid.

Compound (1B) or a salt thereof, i.e., the starting material compound of the present step can be obtained by Process D described later.

As compound (3) or a salt thereof, i.e., the starting material compound of the present step, a commercially available compound is employed or they can be obtained, for example, via a 2-oxo-carbapenam compound according to description of Japanese Patent Application (Kokai) No. Hei 4-330085. Further, it is also included in the present invention that compound (3) is generated from the 2-oxo-carbapenam compound and is employed for the present step without isolating compound (3).

### (Step B2)

The step B2 is to prepare compound (4A) or a salt thereof including (I) a step of removing the hydroxyl or carboxyl protecting group wherein compound (4B) has a hydroxyl or carboxyl protecting group, (II) a step for removing the amino protective group wherein R³ of compound (4B) is an amino protective group, and (III), if necessary, a step of C₁-C₃-alkylating the 1-position of the pyrrolidine ring.

Further, deprotection of the hydroxyl or carboxyl group and deprotection of the amino group can be simultaneously or separately carried out and the order of the deprotection in cases where they are separately carried out is arbitrary.
(I) The deprotection of the hydroxyl or carboxyl group in the present step can be carried out according to the deprotecting step of process A.
(II) The deprotection of the amino group in the present step is accomplished by reacting a compound in which the amino group is protected or a salt thereof with a deprotecting agent in an inert solvent.

The deprotecting agent employed in the present step varies depending on the kind of the amino protective group R³ to be removed and the deprotection can be generally carried out using a known method in this technical field. For example,
(i) wherein R³ is a t-butoxycarbonyl group or a vinyloxycarbonyl group which may be substituted, an acid such as trifluoroacetic acid, hydrogen chloride, hydrogen bromide or sulfuric acid can be employed as a deprotecting agent.
(ii) Wherein R³ is a benzyl group which may be substituted on the aromatic ring or a benzyloxycarbonyl group which may have a substituent on an aromatic ring, the deprotection of the present step can be carried out by a catalytic hydrogenation reaction using a catalyst such as palladium-carbon.
(iii) Wherein R³ is an allyloxycarbonyl group or an allyloxycarbonyl group which may be substituted such as a 2-chloroallyloxycarbonyl group, the deprotection of the present step can be carried out by reacting trimethyl tin hydride and an organic carboxylic acid alkali metal salt such as sodium 2-ethylhexanoate with compound (11) in the presence of a catalytic amount of tetrakis(triphenylphosphine)palladium.
(iv) Wherein R³ is an alkoxycarbonyl group which may be substituted, the deprotection of the present step can be carried out by a reaction with iodotrimethylsilane. Further, the protecting group R³ can be removed by hydrolysis using an acid such as hydrochloric acid and sulfuric acid or a base such as potassium hydroxide and sodium hydroxide.
(v) Wherein R³ is an allyl group which may be substituted at the 2-position, the deprotection of the present step can be carried out by hydrolysis with an acid such as hydrochloric acid after isomerization to an enamine by a rhodium catalyst or the like.

The reaction solvent employed in the present step is not particularly limited so long as it does not inhibit the present reaction, and can be, for example, an ether such as tetrahydrofuran, dioxane or ether; an alcohol such as methanol, ethanol, propanol or isopropyl alcohol; an ester such as methyl acetate, ethyl acetate or isopropyl acetate; water; or a mixed solvent of these, preferably an alcohol, water or a hydrous alcohol.

After completion of the reactions of the above respective deprotection steps, the desired compounds of the respective steps are collected from the reaction mixture according to an ordinary method. For example, after the impurities are removed by filtration from the reaction mixture, the desired compounds can be obtained by distilling off the solvent. The thus obtained desired compounds of the respective steps can be purified, if necessary, by an ordinary method, for example, a recrystallization method, preparative thin layer chromatography, column chromatography or the like. Further, they can be also employed for the subsequent step without purification.
(III) The alkylation in the present step is accomplished by a reductive alkylation reaction after an imine is produced by reacting the compound in which the 1-position of the pyrrolidine ring is not protected with an aldehyde (the corresponding formaldehyde, acetaldehyde or propionaldehyde in the case of methyl, ethyl and propyl), for example, under weak acidic conditions (the reaction system is adjusted to pH 3 to 4 using acetic acid, phosphoric acid buffer or the like).

The aldehyde employed in the present step is formaldehyde in cases where the alkylation of the present step is methylation, it is acetaldehyde in the case of ethylation and it is propylaldehyde in the case of propylation.

The reduction method which can be employed in the present step includes a catalytic hydrogenation reaction using platinum oxide or palladium-carbon as a catalyst and reduction using sodium cyanoborohydride, preferably a catalytic hydrogenation method.

After completion of the reaction of the present step, the desired compound (4A) can be obtained as a crystalline solid of high purity by adding a solvent to the reaction mixture after the catalyst is removed by filtration from the reaction mixture. In particular, in cases where water is employed as the reaction solvent of the present step, after completion of the reaction of the present step, the desired compound (4A) with a high yield and a high quality can be obtained by directly crystallizing it from the reaction mixture from which the catalyst was filtrated without concentrating water and carrying out a troublesome operation such as liquid separation extraction. Further, in cases where only water is employed as the reaction solvent, the by-product derived from the protecting group precipitates in the reaction system and can be also removed simultaneously with the filtration of the catalyst, and it can be expected that the desired compound (4A) of high purity can be efficiently obtained at a high yield only by an extremely easy operation.

The solvent added to the reaction mixture and employed for the crystallization is not particularly limited so long as it does not affect decomposition of compound (4A) and is a solvent in which the compound has a low solubility to some extent, and can be, for example, an alcohol such as methanol, ethanol or isopropyl alcohol; an ether such as tetrahydrofuran or dioxane; a ketone such as acetone; a nitrile such as acetonitrile; or a mixed solvent of these with water, preferably hydrous ethanol, hydrous tetrahydrofuran or hydrous acetone.

Since compound (4A) obtained by the present step has a basic functional group, it can be also obtained as a salt. The acid which forms the salt can be, for example, an inorganic acid such as hydrochloric acid, hydrobromic acid, sulfuric acid, phosphoric acid or carbonic acid; an organic carboxylic acid such as formic acid, acetic acid, oxalic acid or phthalic acid; or an organic sulfonic acid such as methanesulfonic acid or p-toluenesulfonic acid.

Compound (1A) and the salt thereof of the present invention can be prepared by the following Process C.

### (Process C)

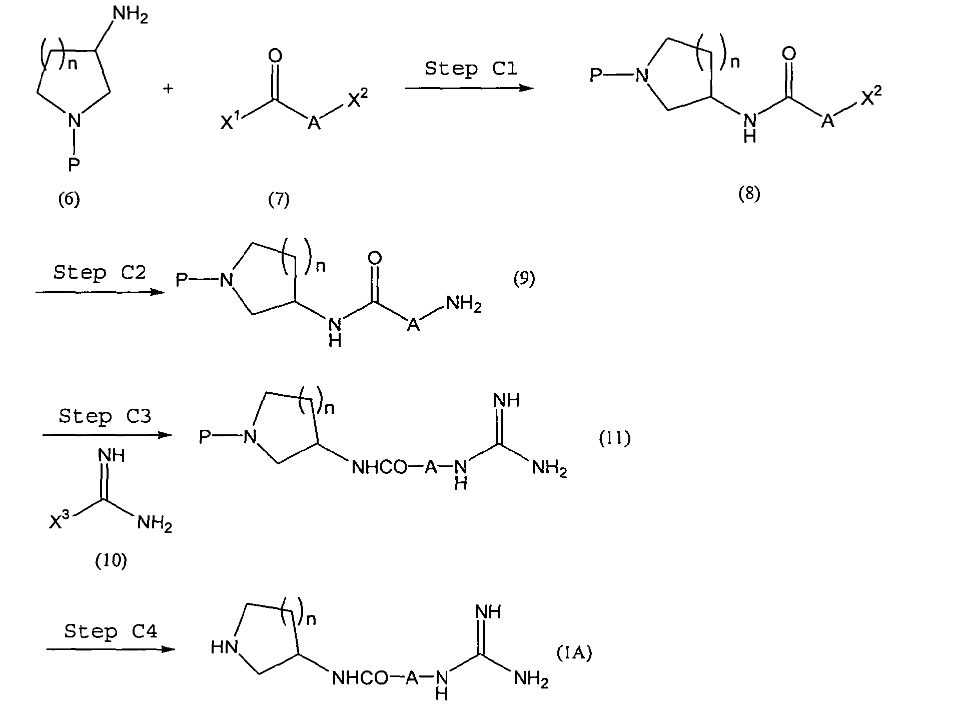

In the above formulae, n, A, P, X¹, X² and X³ have the same meanings as defined above respectively.

### (Step C1)

The step C1 is to prepare compound (8) or the salt thereof and the reaction condition varies depending on the kind of P of compound (6).
(i) In cases where the pyrrolidine moiety of compound (6) becomes a tertiary amine by bonding P, namely, for example, in cases where P is a benzyl group which may be substituted on the aromatic ring or an allyl group which may have a substituent at the 2-position, the present step is accomplished by reacting compound (6) or a salt thereof with compound (7) in an inert solvent, and if necessary, adding a base.
   The reaction solvent employed in the present step is not particularly limited so long as it does not inhibit the reaction, and can be, for example, a nitrile such as acetonitrile; an alcohol such as methanol, ethanol, propanol, isopropyl alcohol or butanol; an ester such as methyl acetate, ethyl acetate or isopropyl acetate; a halogenated hydrocarbon such as dichloromethane, dichloroethane or chloroform; an ether such as ether, dimethoxyethane, tetrahydrofuran or dioxane; an aromatic hydrocarbon such as benzene, toluene or xylene; an amide such as dimethylformamide or dimethylacetamide; a sulfoxide such as dimethyl sulfoxide; or a combination of these solvents at an arbitrary ratio, preferably a nitrile, an ester, a halogenated hydrocarbon or an ether, more preferably acetonitrile or ethyl acetate.
   In cases where the base is employed in the present step, the base employed can be, for example, an organic base such as triethylamine, diisopropylethylamine, 4-methylmorpholine, 4-ethylmorpholine, pyridine, lutidine, 4-dimethylaminopyridine, 1-methylimidazole, 1,2-dimethylimidazole, 1,8-diazabicyclo[5.4.0]-7-undecene (DBU), 1,5-diazabicyclo[4.3.0]non-5-ene (DBN) or 1,4-diazabicyclo[2.2.2]octane (TED); or an inorganic base such as lithium hydroxide, sodium hydroxide, potassium hydroxide, lithium carbonate, sodium carbonate, potassium carbonate, cesium carbonate, sodium hydrogencarbonate or potassium hydrogencarbonate, preferably triethylamine, diisopropylethylamine, sodium carbonate, potassium carbonate or sodium hydrogencarbonate, more preferably sodium hydrogencarbonate.
   The reaction temperature of the present step is usually from 0°C to 50°C, preferably from 0°C to 30°C. The reaction time of the present step varies depending on the reaction temperature and the reaction solvent and is usually from 5 minutes to 10 hours, preferably from 10 minutes to 4 hours.
(ii) In cases where the pyrrolidine moiety of compound (6) becomes a carbamoyl by bonding P, namely, for example, in cases where P is a benzyloxycarbonyl group which may be substituted on the aromatic ring, an alkoxycarbonyl group which may be substituted or an alkenyloxycarbonyl group which may be substituted, the present step is accomplished by reacting compound (6) or a salt thereof with compound (7) in the presence of a base in an inert solvent.

The reaction solvent employed in the present step is similar to those listed in the above (i).

The base employed in the present step can be, for example, an organic base such as triethylamine, diisopropylethylamine, 4-methylmorpholine, 4-ethylmorpholine, pyridine, lutidine, 4-dimethylaminopyridine, 1-methylimidazole, 1,2-dimethylimidazole, 1,8-diazabicyclo[5.4.0]-7-undecene, 1,5-diazabicyclo[4.3.0]non-5-ene or 1,4-diazabicyclo[2.2.2]octane; or an inorganic base such as lithium hydroxide, sodium hydroxide, potassium hydroxide, lithium carbonate, sodium carbonate, potassium carbonate, cesium carbonate, sodium hydrogencarbonate or potassium hydrogencarbonate, preferably triethylamine or diisopropylethylamine.

The reaction temperature of the present step is usually from 0°C to 50°C, preferably from 0°C to 30°C. The reaction time of the present step varies depending on the reaction temperature and the reaction solvent and is usually from 5 minutes to 10 hours, preferably from 10 minutes to 4 hours.

Compound (8) obtained in the present step can be usually used for the subsequent step without isolation and purification.

Further, since compound (8) has an amino group, it can form a salt with an acidic compound. The acidic compound can be, for example, an inorganic acid such as hydrofluoric acid, hydrochloric acid, hydrobromic acid, hydroiodic acid, sulfuric acid, nitric acid, phosphoric acid or carbonic acid; an organic carboxylic acid such as formic acid, acetic acid, trifluoroacetic acid, oxalic acid, phthalic acid or benzoic acid; or an organic sulfonic acid such as methanesulfonic acid, trifluoromethanesulfonic acid, benzenesulfonic acid or p-toluenesulfonic acid.

### (Step C2)

The step C2 is to prepare compound (9) or a salt thereof and is accomplished by reacting a reagent which provides a source of ammonia with compound (8) or a salt thereof in an inert solvent.

The reagent which provides a source of ammonia employed in the present step is not particularly limited so long as it generates ammonia in the system, and can be, for example, ammonium acetate or ammonium carbonate or a combined use thereof with ammonia, in addition to ammonia.

The reaction solvent employed in the present step is not particularly limited so long as it does not inhibit the reaction, and can be, for example, a nitrile such as acetonitrile; an alcohol such as methanol, ethanol, propanol, isopropyl alcohol or butanol; an ester such as methyl acetate, ethyl acetate or isopropyl acetate; a halogenated hydrocarbon such as dichloromethane, dichloroethane or chloroform; an ether such as ether, dimethoxyethane, tetrahydrofuran or dioxane; an aromatic hydrocarbon such as benzene, toluene or xylene; an amide such as dimethylformamide or dimethylacetamide; a sulfoxide such as dimethyl sulfoxide; water; or a combination of these solvents at an arbitrary ratio, preferably a nitrile, an alcohol or a hydrous alcohol, more preferably acetonitrile, methanol or hydrous methanol.

The reaction temperature of the present step is usually from 20°C to 70°C, preferably from 30°C to 50°C. The reaction time of the present step varies depending on the reaction solvent and the reaction temperature and is usually from 2 hours to 24 hours, preferably from 3 hours to 12 hours.

Compound (9) obtained in the present step can be usually used for the subsequent step without isolation and purification.

Since compound (9) has an amino group, it can form a salt with an acidic compound. The acidic compound can be, for example, an inorganic acid such as hydrofluoric acid, hydrochloric acid, hydrobromic acid, hydroiodic acid, sulfuric acid, nitric acid, phosphoric acid or carbonic acid; an organic carboxylic acid such as formic acid, acetic acid, trifluoroacetic acid, oxalic acid, phthalic acid or benzoic acid; or an organic sulfonic acid such as methanesulfonic acid, trifluoromethanesulfonic acid, benzenesulfonic acid or p-toluenesulfonic acid.

### (Step C3)

The step C3 is to prepare compound (11) or a salt thereof and the reaction condition varies depending on the kind of P of compound (9).
(i) In cases where P is a benzyl group which may be substituted on the aromatic ring or an allyl group which may be substituted at the 2-position, the present step is accomplished by reacting compound (9) or a salt thereof with compound (10) or a salt thereof in an inert solvent.
   The reaction solvent employed in the present step is not particularly limited so long as it does not inhibit the reaction, and can be, for example, a nitrile such as acetonitrile; an alcohol such as methanol, ethanol, propanol, isopropyl alcohol or butanol; an ester such as methyl acetate, ethyl acetate or isopropyl acetate; a halogenated hydrocarbon such as dichloromethane, dichloroethane or chloroform; an ether such as ether, dimethoxyethane, tetrahydrofuran or dioxane; an aromatic hydrocarbon such as benzene, toluene or xylene; an amide such as dimethylformamide or dimethylacetamide; a sulfoxide such as dimethyl sulfoxide; water; or a combination of these solvents at an arbitrary ratio, preferably an alcohol, an aromatic hydrocarbon, an amide, a sulfoxide, water or a combination of these solvents at an arbitrary ratio, more preferably butanol, toluene, dimethylformamide, water or a combination of these solvents at an arbitrary ratio.
   The reaction temperature of the present step is usually from 0°C to 100°C, preferably from 20°C to 90°C. The reaction time of the present step varies depending on the reaction temperature, the reaction solvent and the kind of the leaving group of compound (10), and is usually from 30 minutes to 48 hours, preferably from 5 hours to 40 hours.
(ii) In cases where P is a benzyloxycarbonyl group which may be substituted on the aromatic ring, an alkoxycarbonyl group which may be substituted or an alkenyloxycarbonyl group which may be substituted, the present step is accomplished by reacting compound (9) or a salt thereof with compound (10) or a salt thereof in the presence of a base in an inert solvent.

The reaction solvent employed in the present step is similar to those listed in the above (i).

The base employed in the present step can be, for example, an organic base such as triethylamine, diisopropylethylamine, 4-methylmorpholine, 4-ethylmorpholine, pyridine, lutidine, 4-dimethylaminopyridine, 1-methylimidazole, 1,2-dimethylimidazole, 1,8-diazabicyclo[5.4.0]-7-undecene, 1,5-diazabicyclo[4.3.0]non-5-ene or 1,4-diazabicyclo[2.2.2]octane; or an inorganic base such as lithium hydroxide, sodium hydroxide, potassium hydroxide, lithium carbonate, sodium carbonate, potassium carbonate, cesium carbonate, sodium hydrogencarbonate or potassium hydrogencarbonate, preferably triethylamine or diisopropylethylamine.

After completion of the reaction of the present step, a solvent can be added to the reaction mixture to give compound (11) as a crystalline solid of high purity.

The solvent added to the reaction mixture and employed for the crystallization is not particularly limited so long as it does not affect decomposition of compound (11) and is a solvent in which the compound has a low solubility to some extent, and can be, for example, a nitrile such as acetonitrile; an alcohol such as methanol, ethanol, propanol, isopropyl alcohol, butanol or t-butyl alcohol; an ester such as methyl acetate, ethyl acetate or isopropyl acetate; a halogenated hydrocarbon such as dichloromethane, dichloroethane or chloroform; an ether such as ether, dimethoxyethane, tetrahydrofuran or dioxane; a ketone such as acetone or methyl ethyl ketone; an aromatic hydrocarbon such as benzene, toluene or xylene; water; or a combination of these solvents at an arbitrary ratio, preferably an alcohol, an ester, water, a hydrous alcohol, a hydrous ester or a hydrous ketone, more preferably a hydrous alcohol.

Compound (11) obtained by the present step can be usually employed for the subsequent step without isolation and purification.

Further, compound (11) can afford a crystalline solid as a salt, and an acid which forms the salt can be, for example, an inorganic acid such as hydrochloric acid, sulfuric acid, nitric acid or phosphoric acid; an organic carboxylic acid such as formic acid, acetic acid, trifluoroacetic acid, oxalic acid or phthalic acid; or an organic sulfonic acid such as methanesulfonic acid, benzenesulfonic acid, p-toluenesulfonic acid or trifluoromethanesulfonic acid, preferably an inorganic acid, more preferably hydrochloric acid or sulfuric acid.

### (Step C4)

The step C4 is to prepare compound (1A) or a salt thereof by removing the amino protecting group of compound (11) and is accomplished by reacting compound (11) or a salt thereof with a deprotecting agent in an inert solvent. The present step can be carried out according to the deprotection of the amino group of the step B2.

After completion of the reaction of the present step, the desired compound (1A) of the present reaction can be collected from the reaction mixture as a salt with an acidic compound or a free form according to an ordinary method. The salt of compound (1A) is obtained, for example, by collecting by filtration of the precipitated desired compound from the reaction mixture in which an acidic compound is employed as a deprotecting agent. Further, in cases where a catalytic hydrogenation reaction is carried out, an acidic compound is added before or after the reaction, and after the deprotection reaction, an appropriate solvent is employed to precipitate a crystalline solid to give compound (1A) as a crystalline solid of high purity.

The solvent added to the reaction mixture and employed for the crystallization is not particularly limited so long as it does not affect decomposition of compound (1A) and is a solvent in which the compound has a low solubility to some extent, and can be, for example, a nitrile such as acetonitrile; an alcohol such as methanol, ethanol, propanol, isopropyl alcohol, butanol or t-butyl alcohol; an ester such as methyl acetate, ethyl acetate or isopropyl acetate; a halogenated hydrocarbon such as dichloromethane, dichloroethane or chloroform; an ether such as ether, dimethoxyethane, tetrahydrofuran or dioxane; a ketone such as acetone or methyl ethyl ketone; an aromatic hydrocarbon such as benzene, toluene or xylene; an amide such as dimethylformamide or dimethylacetamide; a sulfoxide such as dimethyl sulfoxide; water; or a combination of these solvents at an arbitrary ratio, preferably an alcohol, an ester, an amide, water, a hydrous alcohol, a hydrous ester, a hydrous ketone or a combination of these at an arbitrary ratio, more preferably an alcohol, an amide, water or a combination of these at an arbitrary ratio.

Compound (1B) and a salt thereof of the present invention can be prepared by the following Process D.

### (Process D)

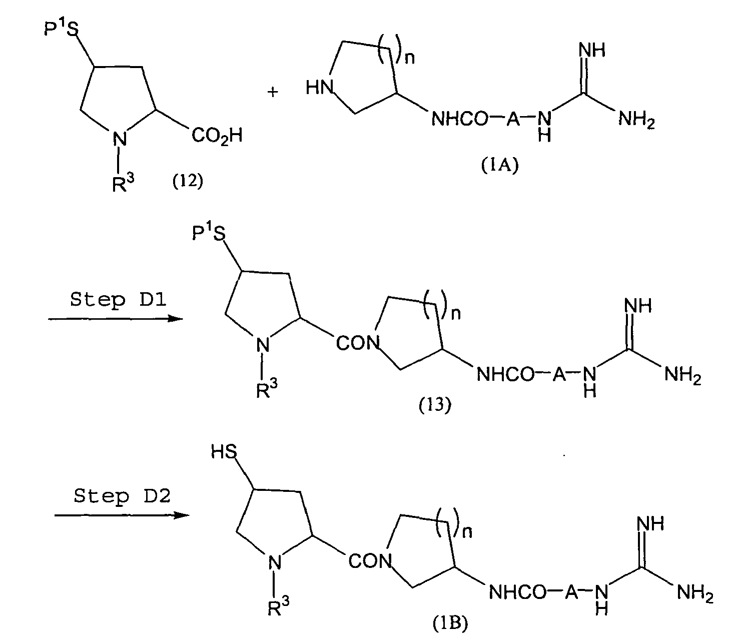

In the above formulae, n, A and R³ have the same meanings as defined above respectively, P¹ represents a mercapto protecting group (the protecting group is, for example, an acyl group which may be substituted or a benzyl group which may be substituted, preferably a benzyl group having an oxygen functional group at the 4-position, more preferably a 4-methoxybenzyl group).

### (Step D1)

The step D1 is to prepare compound (13) by reacting compound (12) with a secondary amine compound (1A) and is accomplished by reacting compound (12) or a salt thereof with compound (1A) or a salt thereof in the presence of a condensing agent and, if necessary, a base in an inert solvent.

The condensing agent employed in the present step is not particularly limited so long as it is usually employed for amidation of a carboxyl group, and can be, for example, N,N'-carbonyldiimidazole; a carbodiimide such as 1,3-dicyclohexylcarbodiimide, N,N'-diisopropylcarbodiimide or 1-(3-dimethylaminopropyl)-3-ethylcarbodiimide hydrochloride; or a phosphoric ester such as diethylphosphoryl cyanide or diphenylphosphoryl azide, preferably N,N'-carbonyldiimidazole.

The base employed in the present step can be, for example, an organic base such as triethylamine, diisopropylethylamine, 4-methylmorpholine, 4-ethylmorpholine, pyridine, lutidine, 4-dimethylaminopyridine, 1-methylimidazole, 1,2-dimethylimidazole, 1,8-diazabicyclo[5.4.0]-7-undecene, 1,5-diazabicyclo[4.3.0]non-5-ene or 1,4-diazabicyclo[2.2.2]octane; or an inorganic base such as lithium hydroxide, sodium hydroxide, potassium hydroxide, lithium carbonate, sodium carbonate, potassium carbonate, cesium carbonate, sodium hydrogencarbonate or potassium hydrogencarbonate, preferably an organic base, more preferably N,N'-diisopropylamine.

The reaction solvent employed in the present step is not particularly limited so long as it does not inhibit the reaction, and can be, for example, a nitrile such as acetonitrile; an ester such as methyl acetate, ethyl acetate or isopropyl acetate; a halogenated hydrocarbon such as dichloromethane, dichloroethane or chloroform; an ether such as ether, dimethoxyethane, tetrahydrofuran or dioxane; an aromatic hydrocarbon such as benzene, toluene or xylene; an amide such as dimethylformamide or dimethylacetamide; a sulfoxide such as dimethyl sulfoxide; or a combination of these solvents at an arbitrary ratio, preferably a nitrile, an ether or an amide, more preferably acetonitrile, tetrahydrofuran or dimethylformamide.

Further, the step D1 is also accomplished by, after compound (12) or a salt thereof is reacted with an acid halide or the like to convert it to an acid anhydride in the presence of a base in an inert solvent, reacting compound (1A) or a salt thereof with the acid anhydride.

The acid halide employed in the present step can include an alkyl halogenated carbonate such as ethyl chlorocarbonate, isopropyl chlorocarbonate or isobutyl chlorocarbonate; and an alkanoyl halide having a branch on the α-carbon such as pivaloyl chloride, preferably pivaloyl chloride.

The base employed in the present step can be, for example, an organic base such as triethylamine, diisopropylethylamine, 4-methylmorpholine, 4-ethylmorpholine, pyridine, lutidine, 4-dimethylaminopyridine, 1-methylimidazole, 1,2-dimethylimidazole, 1,8-diazabicyclo[5.4.0]-7-undecene, 1,5-diazabicyclo[4.3.0]non-5-ene or 1,4-diazabicyclo[2.2.2]octane; or an inorganic base such as lithium hydroxide, sodium hydroxide, potassium hydroxide, lithium carbonate, sodium carbonate, potassium carbonate, cesium carbonate, sodium hydrogencarbonate or potassium hydrogencarbonate, preferably an organic base, more preferably N,N'-diisopropylamine.

The reaction solvent employed in the present step is not particularly limited so long as it does not inhibit the reaction, and can be, for example, a nitrile such as acetonitrile; an ester such as methyl acetate, ethyl acetate or isopropyl acetate; a halogenated hydrocarbon such as dichloromethane, dichloroethane or chloroform; an ether such as ether, dimethoxyethane, tetrahydrofuran or dioxane; an aromatic hydrocarbon such as benzene, toluene or xylene; an amide such as dimethylformamide or dimethylacetamide; a sulfoxide such as dimethyl sulfoxide; or a combination of these solvents at an arbitrary ratio, preferably a nitrile, an ether or an amide, more preferably acetonitrile, tetrahydrofuran or dimethylformamide.

The reaction temperature of the present step is usually from 0°C to 80°C, preferably from 5°C to 50°C. The reaction time of the present step varies depending on the reaction solvent and the reaction temperature and is usually from 30 minutes to 48 hours, preferably from 3 hours to 24 hours. In cases where an acid halide is employed, conversion to the acid anhydride is usually from 5 minutes to 2 hours, preferably from 10 minutes to 1 hour and the reaction after that is usually from 1 hour to 48 hours, preferably from 2 hours to 24 hours.

After completion of the reaction, the desired compound (13) of the present step is collected from the reaction mixture according to an ordinary method. It can be obtained, for example, by adding an organic solvent immiscible with water to the reaction mixture liquid or a residue obtained by distilling off the solvent of the reaction mixture liquid and after washing it with water, distilling off the solvent. The obtained desired compound can be further purified, if necessary, by an ordinary method, for example, recrystallization, reprecipitation or chromatography. Further, the desired compound (13) can be also used for the subsequent step without isolation and purification.

Since compound (13) obtained by the present step has a basic functional group, it can be also obtained as a salt. The acid which forms the salt can be, for example, an inorganic acid such as hydrochloric acid, hydrobromic acid, sulfuric acid, phosphoric acid or carbonic acid; an organic carboxylic acid such as formic acid, acetic acid, oxalic acid or phthalic acid; or an organic sulfonic acid such as methanesulfonic acid or p-toluenesulfonic acids.

### (Step D2)

The step D2 is to prepare compound (1B) and is accomplished by reacting compound (13) or a salt thereof with a deprotecting agent of a thiol group in an inert solvent. The deprotecting agent employed in the present step varies depending on the kind of the protecting group to be removed but the present step is generally carried out using a known method in this technical field.

In cases where P¹ is an acyl group which may be substituted, the present step is accomplished by a general hydrolysis reaction in the presence of alcohol or water.

The deprotecting agent employed in the present step is an alkali metal hydroxide such as sodium hydroxide and potassium hydroxide; or an alkali metal alcolate such as sodium methylate and sodium ethylate, preferably sodium hydroxide or sodium methylate.

The reaction solvent employed in the present step is not particularly limited so long as it does not inhibit the reaction, and can be water; an alcohol such as methanol, ethanol, 1-propanol, butanol or isopropanol; an ether such as ether, dimethoxyethane, tetrahydrofuran or dioxane; an aromatic hydrocarbon such as benzene, toluene or xylene; or a combination of these at an arbitrary ratio, preferably an alcohol, more preferably methanol or ethanol.

The reaction temperature of the present step is usually from 0°C to 80°C, preferably from 5°C to 40°C. The reaction time of the present step varies depending on the reaction solvent and the reaction temperature and is usually from 10 minutes to 5 hours, preferably from 30 minutes to 2 hours.

In cases where P¹ is a benzyl group having an oxygen functional group at the 4-position which may be substituted, the present step is carried out in an anisole solvent using an acid.

The acid employed in the present step is an inorganic acid such as hydrogen fluoride, mercury(II) trifluoroacetate or trifluoroacetic acid/trifluoromethanesulfonic acid, preferably trifluoroacetic acid/trifluoromethanesulfonic acid.

The reaction temperature of the present step is usually from 0°C to 80°C, preferably from 5°C to 40°C. The reaction time of the present step varies depending on the reaction solvent and the reaction temperature and is usually from 10 minutes to 48 hours, preferably from 30 minutes to 12 hours.

After completion of the reaction, the desired compound (1B) of the present step is collected from the reaction mixture according to an ordinary method. It can be obtained, for example, by adding an organic solvent immiscible with water to the reaction mixture liquid or the residue obtained by distilling off the solvent of the reaction mixture liquid and after washing it with water, distilling off the solvent. The obtained desired compound can be further purified, if necessary, by an ordinary method, for example, recrystallization, reprecipitation or chromatography. Further, the desired compound (1B) can be also used for the subsequent step without isolation and purification.

Since compound (1B) has a basic functional group, it can be also obtained as a salt. The acid which forms the salt can be, for example, an inorganic acid such as hydrochloric acid, hydrobromic acid, sulfuric acid, phosphoric acid or carbonic acid; an organic carboxylic acid such as formic acid, acetic acid, oxalic acid or phthalic acid; or an organic sulfonic acid such as methanesulfonic acid or p-toluenesulfonic acid.

### [Examples]

In the following, the present invention is described in more detail with reference to Examples and Reference Examples but the present invention is not limited thereto.

### Example 1

4-Nitrobenzyl (1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylate_{·}hydrochloride (Step A1)

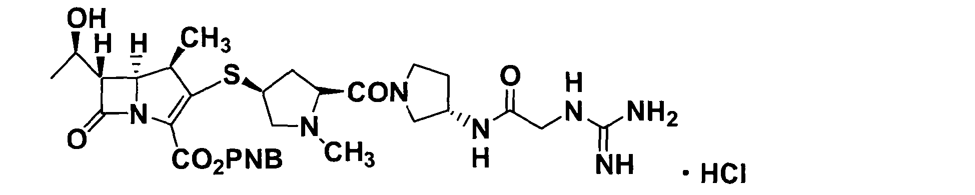

In the above formula, PNB represents a p-nitrobenzyl group, i.e., a carboxyl protecting group (hereinafter the same shall apply).

(2S,4S)-5-Methyl-2-thia-5-azabicyclo[2.2.1]heptan-3-one-hydrochloride (30.00 g) obtained by a method described in Japanese Patent Application (Kokai) No. 2002-212183 was dissolved in dimethyl sulfoxide (240 mL), and sodium hydrogencarbonate (28.06 g) was added to this solution, followed by stirring of the mixture at room temperature under a nitrogen atmosphere for 30 minutes. (S)-[3-(2-guanidino)acetylamino]pyrrolidine-sulfate (52.04 g) obtained in Example 11 was added to the reaction mixture and after the mixture was stirred at 40°C for 2.5 hours, the reaction mixture was cooled to room temperature and 4-nitrobenzyl (1R,5S,6S)-6-[(1R)-1-hydroxyethyl]-2-[(diphenylphosphino)oxy]-1-methyl-carbapen-2-em-3-carboxylate (94.32 g) and dimethyl sulfoxide (60 mL) were added thereto. After diisopropylethylamine (21.58 g) was further added dropwise to the reaction mixture, the mixture was stirred at room temperature for 1.5 hours. Thereafter, after 2-propanol (600 mL) and ethyl acetate (1.8 L) were added to the reaction mixture and the mixture was stirred for 3 hours, ethyl acetate (600 mL) was further added to the reaction mixture and the mixture was stirred for 21 hours. The crystalline solid precipitated from the reaction mixture was collected by filtration and washed with 2-propanol (300 mL) to give a crude crystalline solid of the title compound.

The crude crystalline solid was added to water (900 mL) under ice-cooling and after the mixture was stirred at the same temperature for 1 hour, a crystalline solid was collected by filtration. The crystalline solid was washed with ice water (600 mL) and dried under reduced pressure at room temperature to give crystals (98.84 g) of the title compound. Yield: 87.8%, purity: 95%.

IR absorption spectrum (KBr) ν max (cm⁻¹) : 3283, 3250, 3069, 2971, 2875, 2792, 1767, 1677, 1548, 1521, 1454, 1378, 1341, 1295, 1282, 1240, 1209, 1181, 1142, 1108, 1077, 1055, 1014, 990, 958, 938, 927, 912, 847, 799, 766, 739, 691, 609, 580, 554.

NMR spectrum (400 MHz, DMSO) δ ppm : 1.15 (6H, d, J=6Hz), 1.60-1.67 (1H, m), 1.73-1.88 (1H, m), 1.95-2.10 (1H, m), 2.21 (3H, d, J=8Hz), 2.54-2.66 (1H, m), 2.72 (1H, dd, J=7, 10Hz), 2.91-2.96 (1H, m), 3.06-3.15 (1H, td, J=8, 18Hz), 3.21-3.65 (4H, m), 3.72-3.81 (3H, m), 3.93-3.99 (1H ,m), 4.20 (1H, dd, J=2, 9Hz), 4.20-4.31 (1H, m), 5.09 (1H, d, J=5Hz), 5.44 (2H, dd, J=14, 62Hz), 7.33 (3H, b), 7.72 (2H, d, J=8Hz), 8.23 (2H, d, J=8Hz), 8.53 (1H, d, J=7Hz).

### Example 2

4-Nitrobenzyl (1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylate-hydrochloride (Step A1)

(S)-[3-(2-Guanidino)acetylamino]pyrrolidine-hydrochloride (23.71 g) obtained in Example 10 was dissolved in a dimethyl sulfoxide solution (240 mL), and (2S,4S)-5-methyl-2-thia-5-azabicyclo[2.2.1]heptan-3-one·hydrochloride (15.00 g) obtained by a method described in Japanese Patent Application (Kokai) No. 2002-212183 and sodium hydrogencarbonate (21.04 g) were added to this solution. The interior of the reaction system was adjusted to slightly reduced pressure and the mixture was stirred at room temperature for 5.5 hours. Thereafter, 4-nitrobenzyl (1R,5S,6S)-6-[(1R)-1-hydroxyethyl]-2-[(diphenylphosphino)oxy]-1-methyl-carbapen-2-em-3-carboxylate (48.89 g), dimethyl sulfoxide (30 mL) and diisopropylethylamine (2.88 mL) were added to the reaction mixture, and the mixture was stirred at room temperature for 4.5 hours. Thereafter, 2-propanol (30 mL) and ethyl acetate (900 mL) were added to the reaction mixture, and the mixture was stirred at room temperature for 13 hours. Further, ethyl acetate (600 mL) was added thereto, and the mixture was stirred for 9 hours. The precipitated crystalline solid was collected by filtration and washed with 2-propanol (300 mL) to give crude crystalline solid of the title compound.

The crude crystalline solid (89.58 g) was added to water (450 mL) under ice-cooling and after the mixture was stirred at the same temperature for 1 hour, a crystalline solid was collected by filtration, washed with ice water (225 mL) and dried under reduced pressure at room temperature to give crystals (49.16 g) of the title compound. Yield: 84.3%, purity: 96%.

### Example 3

4-Nitrobenzyl (1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylate·hydrochloride (Step A1)

(S)-[3-(2-Guanidino)acetylamino]pyrrolidine-hydrochloride (4.75 g) obtained in Example 10 was dissolved in dimethyl sulfoxide (54 mL), and (2S,4S)-5-methyl-2-thia-5-azabicyclo[2.2.1]heptan-3-one-hydrochloride (3.00 g) obtained by a method described in Japanese Patent Application (Kokai) No. 2002-212183 and sodium hydrogencarbonate (4.21 g) were added to this solution, followed by stirring of the mixture at room temperature for 6 hours. Thereafter, 4-nitrobenzyl (1R,5S,6S)-6-[(1R)-1-hydroxyethyl]-2-[(diphenylphosphino)oxy]-1-methyl-carbapen-2-em-3-carboxylate (9.75 g) and dimethyl sulfoxide (6 mL) were added to the reaction mixture, and the mixture was stirred at room temperature for 4 hours. Thereafter, acetone (120 mL) and 1% brine (240 mL) were successively added to the reaction mixture, and the mixture was stirred at room temperature for 13 hours. The precipitated crystalline solid was collected by filtration, washed with acetone (20 mL) and 1% brine (40 mL), then washed with ice water (27 mL) and dried under reduced pressure to give the title compound (16.67 g). Yield: 91.7%, purity: 97%.

### Example 4

(1R,5S,6S)-6-[(1R)-1-Hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylic acid (Deprotecting Step)

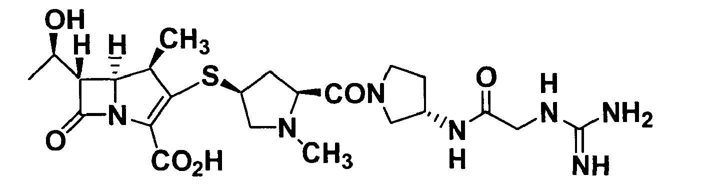

4-Nitrobenzyl (1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylate-hydrochloride (3.00 g) obtained in Example 2 and sodium hydrogencarbonate (360 mg) were suspended in purified water (30 mL), and 7.5% palladium-carbon (1.92 g) was added to the suspension, followed by stirring of the mixture at 15°C under a hydrogen atmosphere for 5 hours. After the reaction, the catalyst was filtrated off (the catalyst was washed with purified water (5 mL)) and activated carbon (150 mg) was added to the filtrate. After the mixture was stirred at 15°C for 0.5 hours, the activated carbon was separated by filtration and washed with purified water (6 mL). Ethanol (20 mL) was added to the filtrate and a seed crystal was added thereto. After the mixture was stirred for 2 hours, ethanol (100 mL) was further added to the reaction mixture. The mixture was stirred for 0.5 hours and cooled to 5°C. After the mixture was stirred at the same temperature for 0.5 hours, the precipitated crystalline solid was collected by filtration. After it was washed with a 75% aqueous ethanol solution, it was dried under reduced pressure to give the title compound (1.86 g). Yield: 81.8%, purity: 100% (dehydration/desolvation conversion value).

IR absorption spectrum (KBr) ν max (cm⁻¹): 3409, 3345, 3275, 3185, 2967, 2884, 1761, 1674, 1644, 1586, 1551, 1452, 1415, 1380, 1369, 1340, 1282, 1254.

NMR spectrum (400 MHz, DMSO) δ ppm : 1.13-1.24 (4.5H, m), 1.30 (3H, d, J=6.4Hz), 1.57-1.72 (1H, m), 1.93-2.10 (1H, m), 2.15-2.35 (1H, m), 2.27,2.29 (3H, sX2), 2.68-2.88 (2H, m), 3.09 (1H, d, J=10.6Hz), 3.29-3.73 (7H, m), 3.75-3.93 (2H, m), 4.01 (2H, s), 4.12-4.30 (2H, m), 4.38-4.50 (1H, m).

### Example 5

(1R,5S,6S)-6-[(1R)-1-Hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylic acid (Deprotecting Step)

7.5% Palladium-carbon (1.72 g) was added to a suspension of 4-nitrobenzyl (1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylate-hydrochloride (3.0 g) obtained in Example 3 and sodium hydrogencarbonate (355 mg) in purified water (30 mL) was added, and the mixture was stirred at 15°C under a hydrogen atmosphere for 3 hours. After the reaction, the catalyst was filtrated (the catalyst was washed with purified water (6 mL)). Activated carbon (150 mg) was added to the filtrate and after the mixture was stirred at room temperature (20°C) for 0.5 hours, the activated carbon was separated by filtration and washed with purified water (3 mL). Acetone (60 mL) was added thereto and a seed crystal was added thereto, followed by further addition of acetone (60 mL). After the mixture was stirred for 1 hour, it was left to stand at 5°C overnight. The precipitated crystalline solid was collected by filtration and it was washed with a 75% aqueous acetone solution, followed by drying under reduced pressure to give the title compound (1.91 g). Yield: 84.0%, purity: 100% (dehydration/desolvation conversion value).

### Example 6

(1R,5S,6S)-6-[(1R)-1-Hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylic acid (Deprotecting Step)

4-Nitrobenzyl (1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylate-hydrochloride (3.0 g) obtained in Example 2 was suspended in purified water (30 mL), and the suspension was charged in a pressure-resistant container of 100 mL. 7.5% Palladium-carbon (0.58 g) was added to the suspension, and the mixture was stirred at 30°C under a hydrogen atmosphere (3.0 kg/cm²) for 3 hours. After the reaction, sodium hydrogencarbonate (0.36 g) was added to the reaction mixture and after the mixture was stirred for 1 hour, the catalyst was filtered off (the catalyst was washed with purified water (6 mL)). Zeolite (NaX type, 0.90 g) was added to the filtrate and after the mixture was stirred at room temperature for 1 hour, the zeolite was filtered off. After ethanol (18 mL) was added to the filtrate, a seed crystal was added thereto and ethanol (90 mL) was further added thereto. The mixture was stirred for 2 hours and left to stand at from 0°C to 5°C overnight. The precipitated crystalline solid was collected by filtration and washed with a 75% aqueous ethanol solution, followed by drying under reduced pressure to give the title compound (1.54 g). Yield: 72.3%, purity: 99% (dehydration/desolvation conversion value).

### Example 7

(1R,5S,6S)-6-[(1R)-1-Hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylic acid (Deprotecting Step)

4-Nitrobenzyl (1R,5S,6S)-6-[(1R)-1-hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylate·hydrochloride (3.0 g) obtained in Example 2 was suspended in purified water (30 mL), and 5% palladium zeolite (0.75 g) was added to the suspension, followed by stirring of the mixture at 30°C under a hydrogen atmosphere (3 kg/cm²) for 5 hours. After the reaction, sodium hydrogencarbonate (0.36 g) was added to the reaction mixture and after the mixture was stirred for 1 hour, the catalyst was filtered off (the catalyst was washed with purified water (6 mL)). After ethanol (18 mL) was added to the filtrate, a seed crystal was added thereto and ethanol (90 mL) was further added thereto, followed by stirring of the mixture for 2 hours. The precipitated crystalline solid was collected by filtration and washed with a 75% aqueous ethanol solution, followed by drying under reduced pressure to give the title compound (1.37 g). Yield: 62.8%, purity: 96% (dehydration/desolvation conversion value).

### Example 8

(1R,5S,6S)-6-[(1R)-1-Hydroxyethyl]-1-methyl-2-[(2S,4S)-2-[(3S)-3-(2-guanidinoacetylamino)pyrrolidin-1-ylcarbonyl]-1-methylpyrrolidin-4-ylthio]-1-carbapen-2-em-3-carboxylic acid (Deprotecting Step)

4-Nitrobenzyl (1R,5S,6S)-6-[(1R)-1-hydroxyethyl]-1-methyl-2-[(2S, 4S)-2-[(3S)-3-(2-guanidinoacetylamino)pyrrolidin-1-ylcarbonyl]-1-methylpyrrolidin-4-ylthio]-1-carbapen-2-em-3-carboxylate·hydrochloride (3.0 g) obtained in Example 2 and sodium hydrogencarbonate (0.36 g) were suspended in purified water (30 mL), and 7.5% palladium-carbon (1.73 g) was added to the suspension, followed by stirring of the mixture at 20°C under a hydrogen atmosphere (3.0 kg/cm²) for 3 hours. After the reaction, the catalyst was filtered off (the catalyst was washed with purified water (6 mL)). Ethanol (18 mL) was added to the filtrate and a seed crystal was added thereto, followed by further addition of ethanol (90 mL). After the mixture was stirred for 2 hours, it was left to stand at from 0°C to 5°C overnight. The precipitated crystalline solid was collected by filtration and after it was washed with a 75% aqueous ethanol solution, it was dried under reduced pressure to give the title compound (1.93 g). Yield: 84.9%, purity: 98% (dehydration/desolvation conversion value).

### Example 9

(S)-[3-(2-Guanidino)acetylamino]pyrrolidine·hydrochloride (Steps C1 to C4)

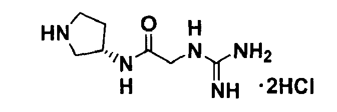

(S)-3-Amino-1-benzylpyrrolidine (20.00 g) was dissolved in acetonitrile (80 mL) and a solution of chloroacetyl chloride (13.50 g) in acetonitrile (20 mL) was added dropwise to the solution under ice-cooling, followed by stirring of the mixture at the same temperature for 1 hour (Step C1). Thereafter, 28% ammonia water (600 mL) was poured into the reaction mixture and the temperature of the mixture was raised to 40°C, followed by stirring of the mixture at the same temperature for 3 hours. Sodium chloride (60 g) was added to the reaction mixture and the mixture was extracted with a solvent mixture of tetrahydrofuran/toluene (3/1, 400 mL) three times. After the organic layer was concentrated to 60 mL under reduced pressure, butanol (100 mL) was poured into the concentrated liquid and it was concentrated to 60 mL under reduced pressure again (Step C2). Butanol (40 mL) was added to the concentrated substance and 1H-pyrazole-1-carboxamidine hydrochloride (16.60 g) was added thereto, followed by stirring of the mixture at room temperature for 6 hours. A solvent mixture of butanol/toluene (7/3, 100 mL) was added to the reaction mixture and the mixture was extracted with water (100 mL) three times. After the aqueous layers were combined and concentrated hydrochloric acid (6.20 g) was added thereto, the mixture was concentrated to 120 mL under reduced pressure (Step C3). After concentrated hydrochloric acid was added to the concentrated liquid to adjust the pH to 4, it was diluted with ethanol (120 mL) and 5% palladium-carbon (4.00 g) was added thereto, followed by stirring of the mixture at 50°C under a hydrogen atmosphere for 3 hours. After the reaction, the catalyst was filtered off and the obtained filtrate was concentrated to 44 mL under reduced pressure. Dimethylformamide (100 mL) and concentrated hydrochloric acid (12.00 g) were added to the concentrated liquid and after isopropyl alcohol was added dropwise thereto at from 45 to 50°C, a seed crystal was added thereto and the mixture was stirred at the same temperature for 1 hour. Further, isopropyl alcohol (200 mL) was added dropwise thereto and the mixture was cooled to 30°C or lower, followed by stirring of the mixture at the same temperature for 30 minutes and further stirring under ice-cooling for 1 hour. The precipitated crystalline solid was collected by filtration and washed with isopropyl alcohol (100 mL), followed by drying under reduced pressure to give the title compound (24.36 g). Yield: 83.2%

IR absorption spectrum (KBr) ν max(cm⁻¹) : 3373, 3312, 3270, 3208, 3154, 3057, 3027, 2915, 1670, 1645, 1613, 1586, 1574, 1552, 1441, 1351, 1273, 660, 623, 578.

NMR spectrum (400 MHz. D₂O) δ ppm : 1.90 (1H, td, J=14,7Hz), 2.23 (1H, td, J=14, 7Hz), 3.14 (1H, dd, J=5, 13Hz), 3.23-3.39 (2H, m), 3.43 (1H, dd, J=7, 13Hz), 3.86 (2H, s), 4.32-4.39 (1H, m).

### Example 10

### (S)-[3-(2-Guanidino)acetylamino]pyrrolidine·hydrochloride (Steps C1 to C4)

(S)-3-Amino-1-tert-butyloxycarbonylpyrrolidine (200.00 g) was dissolved in ethyl acetate (2000 mL) and after triethylamine (108.66 g) was added to the solution under ice-cooling, chloroacetyl chloride (121.28 g) was further added dropwise thereto and the mixture was stirred at the same temperature for 10 minutes. After the reaction, the reaction mixture was successively washed with a 5% aqueous sodium hydrogencarbonate solution (1000 mL) and 0.5 mol/L hydrochloric acid (1000 mL). After the mixture was concentrated to 400 mL under reduced pressure, operations for adding methanol (1000 mL) thereto and concentrating it to 400 mL under reduced pressure were repeated twice (Step C1). After water (1320 mL) was poured into 28% ammonia water (6200 mL), the above methanol concentrated liquid was added dropwise to the solution. After the mixture was stirred at from 40°C to 45°C for 3.5 hours, the reaction mixture was cooled to room temperature and triethylamine (108.66 g) was added thereto, followed by further stirring of the mixture for 30 minutes. After the reaction mixture was extracted with a solvent mixture of butanol/toluene (1/1, 2400 mL) twice and with the same solvent mixture (4800 mL) once, the organic layers were combined and concentrated to 700 mL under reduced pressure (Step C2). Triethylamine (32.60 g) and 1H-pyrazole-1-carboxamidine hydrochloride (157.37 g) were successively added to the concentrated liquid, and the mixture was stirred at room temperature for 23 hours. The reaction mixture was diluted with a solvent mixture of butanol/toluene (7/3, 1000 mL) and extracted with water (1000 mL) three times. The aqueous layers were combined and concentrated to 500 mL under reduced pressure (Step C3). The concentrated liquid was diluted with isopropyl alcohol (500 mL) and concentrated hydrochloric acid (223.72 g) was added dropwise thereto at room temperature, followed by stirring of the mixture at from 45 to 50°C for 1.5 hours. Thereafter, dimethylformamide (100 mL) was added to the reaction mixture and isopropyl alcohol (4500 mL) was added dropwise thereto, followed by stirring of the mixture at the same temperature for 20 minutes. Further, isopropyl alcohol (200 mL) was poured into the reaction mixture and after the mixture was stirred at the same temperature for 20 minutes, it was cooled to 0°C. After the precipitated crystalline solid was collected by filtration and washed with isopropyl alcohol (1000 mL), it was dried under reduced pressure to give the title compound (232.63 g). Yield: 83.9%.

### Example 11

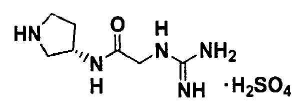

### (S)-[3-(2-Guanidino)acetylamino]pyrrolidine-sulfate

### (1) (S)-[3-(2-Guanidino)acetylamino]-1-benzylpyrrolidine sulfate (Steps C1 to C3)

(S)-3-Amino-1-benzylpyrrolidine (5.00 g) was dissolved in acetonitrile (20 mL) and a solution of chloroacetyl chloride (3.36 g) in acetonitrile (5 mL) was added dropwise to the solution under ice-cooling, followed by stirring of the mixture at the same temperature for 1 hour (Step C1). 25% Ammonia water (150 mL) was added to the reaction mixture and the mixture was stirred at 40°C for 4 hours. The reaction mixture was cooled to 20°C and sodium chloride (15.00 g), butanol (25 mL) and toluene (25 mL) were added thereto, followed by liquid separation extraction. Thereafter, liquid separation extraction was further carried out three times using butanol (25 mL) and toluene (25 mL). The extracts were combined and concentrated to 20 mL under reduced pressure. Dimethylacetamide (13 mL) was added thereto and the mixture was concentrated to 20 mL under reduced pressure again (Step C2). S-methylisothiourea (3.95 g) was added to the concentrated liquid and the mixture was stirred at 80°C for 12 hours. The reaction mixture was cooled to room temperature and after water (10 mL) was added thereto, 28% sulfuric acid (10 mL) and methanol (100 mL) were added dropwise thereto and the mixture was stirred for 2 hours. After the reaction mixture was stirred under ice-cooling for 15 hours, the precipitated crystalline solid was collected by filtration and washed with a solvent mixture of methanol/water (85/15, 20 mL), followed by drying under reduced pressure to give the title compound (8.66 g) (Step C3).

IR absorption spectrum (KBr) ν max(cm⁻¹) : 3410, 3314, 3137, 2993, 2736, 2718, 1698, 1673, 1632, 1608, 1543, 1134, 1120, 620.

NMR spectrum (400 MHz. DMSO) δ ppm : 1.58-1.66 (1H, m), 2.09-2.18 (1H, m), 2.43 (2H, m), 2.71-2.77 (2H, m), 3.67 (2H, s), 3.76 (2H, d, J=6Hz), 4.15-4.25 (1H, m), 7.0-7.32 (8H, m), 7.53-7.56 (1H, m), 8.41 (1H, d, J=7Hz).

### (2) (S)-[3-(2-Guanidino)acetylamino]pyrrolidine-sulfate (Step C4)

(S)-[3-(2-Guanidino)acetylamino]-1-benzylpyrrolidine sulfate (7.00 g) obtained in (1) was suspended in water (70 mL) and 5% palladium-carbon (2.80 g) was added to the suspension, followed by stirring of the mixture at 50°C under a hydrogen atmosphere for 5 hours. After the reaction, the catalyst was filtered off (the catalyst was washed with water (42 mL)) and methanol (112 mL) was added dropwise to the reaction mixture at room temperature. After the mixture was stirred for 1 hour, it was stirred under ice-cooling for 15 hours. After the precipitated crystalline solid was collected by filtration and washed with a solvent mixture of methanol/water (1/1, 28 mL), it was dried under reduced pressure to give the title compound (4.80 g). Yield: 59.7%.

IR absorption spectrum (KBr) ν max(cm⁻¹) : 3373, 3271, 3208, 3154, 3057, 3027, 1670, 1645, 1613, 1586, 1575, 1552, 1351, 1273, 1130, 1120, 620.

NMR spectrum (400 MHz. DMSO) δ ppm : 2.05-2.13 (1H, m), 2.33-2.42 (1H, m), 3.33-3.60 (4H, m), 4.04 (2H, s), 4.51-4.56 (1H, m).

### Example 12

### (S)-[3-(2-Guanidino)acetylamino]pyrrolidine-sulfate

(S)-3-Amino-1-tert-butyloxycarbonylpyrrolidine (5.00 g) was dissolved in ethyl acetate (60 mL) and after triethylamine (2.72 g) was added to the solution under ice-cooling, chloroacetyl chloride (3.03 g) was further added dropwise thereto. After the mixture was stirred at the same temperature for 10 minutes, the reaction mixture was successively washed with a 5% aqueous sodium hydrogencarbonate solution (25 mL) and 0.5 mol/L hydrochloric acid solution and concentrated to 10 mL. Thereafter, methanol (15 mL) was added thereto and an operation for concentrating it to 10 mL was repeated twice.

After water (20 mL) and 28% ammonia water (75 mL) were poured onto ammonium carbonate (25.80 g), the methanol concentrated liquid obtained by the above operation was added dropwise to the solution and the mixture was washed with methanol (38 mL). After the mixture was stirred at 40°C for 10 hours, the reaction mixture was cooled to room temperature and extracted with a solvent mixture of isopropanol/ethyl acetate (1/2, 150 mL) twice. The organic layers were combined and concentrated to 15 mL under reduced pressure. 25 mL of tap water were added to the concentrated substance and after it was concentrated to 15 mL again, 10 mL of water was added thereto. After triethylamine (2.72 g) was added to the solution at room temperature, 1H-pyrazole-1-carboxamidine hydrochloride (3.93 g) was added thereto and the mixture was stirred at room temperature for 40 hours. Thereafter, after the reaction mixture was washed with ethyl acetate (50 mL) five times, concentrated sulfuric acid (5.27 g) was added dropwise to the aqueous layer and the mixture was stirred at room temperature for 2 hours. After ethanol (25 mL) was poured into the reaction mixture, a seed crystal was added thereto and the mixture was stirred at the same temperature for 1.5 hours. After ethanol (25 mL) was added dropwise to the obtained slurry liquid and the mixture was stirred at room temperature for 1 hour, it was ice-cooled and stirred for 30 minutes. After the precipitated crystalline solid was filtered and washed with a solvent mixture of ethanol/water (2/1, 15 mL), it was dried under reduced pressure to give the title compound (5.50 g). Yield: 72.3%.

### Industrial Applicability

By the present invention, 1-methylcarbapenem-type antibacterial agents having a 1-alkylpyrrolidine structure and a guanidyl group which exhibit excellent antibacterial activity can be prepared efficiently and on a large scale.

## Claims

1. A compound or a salt thereof represented by the formula: 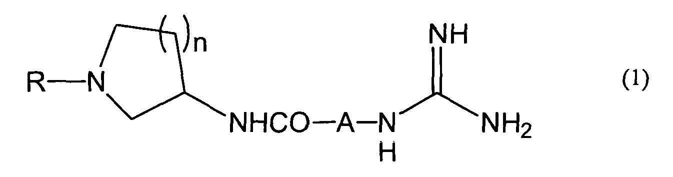 [wherein R represents a hydrogen atom or a group represented by the formula: 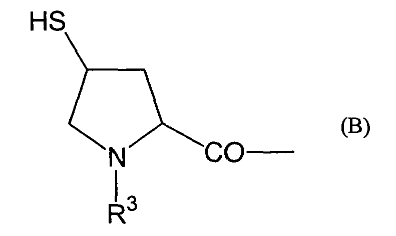 (wherein R³ represents a hydrogen atom, a C₁-C₃ alkyl group or an amino protecting group), n represents 0, 1 or 2 and A represents a C₁-C₃ alkylene group].

2. A compound or a salt thereof according to claim 1, wherein R³ is a C₁-C₃ alkyl group.

3. A compound or a salt thereof according to claim 1, wherein R is a hydrogen atom.

4. A compound or a salt thereof according to any one of claims 1 to 3, wherein n is 0 or 1.

5. A compound or a salt thereof according to any one of claims 1 to 3, wherein n is 1.

6. A compound or a salt thereof according to any one of claims 1 to 5, wherein A is a methylene group.

7. A process for the preparation of a compound or a salt thereof represented by the formula: 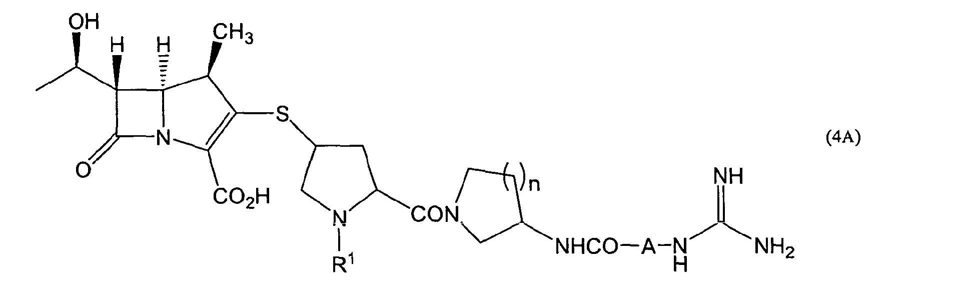 (wherein R¹ represents a C₁-C₃ alkyl group, n reperesents 0, 1 or 2 and A represents a C₁-C₃ alkylene group) comprising a step for preparing a compound or a salt thereof represented by the formula: 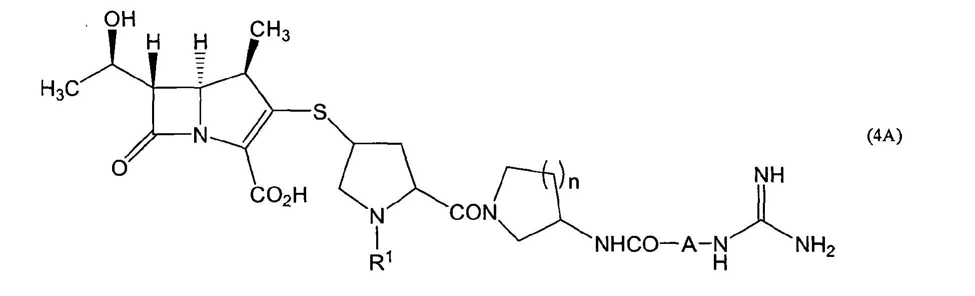 (wherein n, A and R¹ have the same meanings as defined above respectively and the hydroxyl or carboxyl groups can independently be protected) by reacting a compound or a salt thereof represented by the formula: 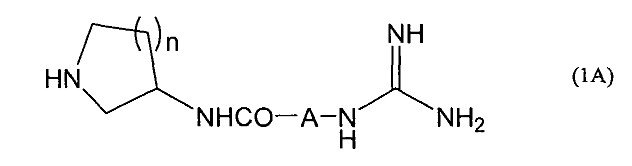 (wherein n and A have the same meanings as defined above respectively), a compound or a salt thereof represented by the formula: 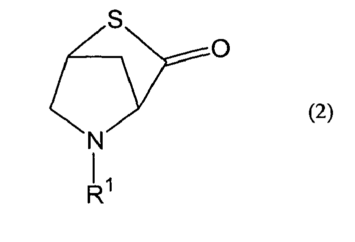 (wherein R¹ has the same meaning as defined above), and a compound or a salt thereof represented by the formula: 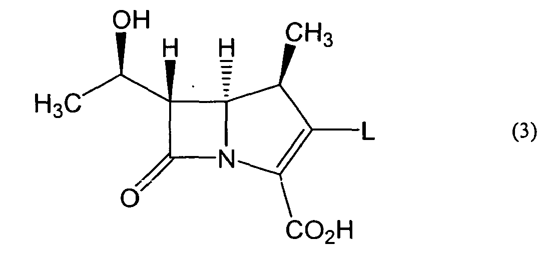 (wherein L represents a leaving group and the hydroxyl or carboxyl groups can independently be protected) in the presence of a base in an inert solvent.

8. The production process according to claim 7, wherein R¹ is a methyl group.

9. The production process according to claim 7 or 8, wherein n is 0 or 1.

10. The production process according to claim 7 or 8, wherein n is 1.

11. The production process according to any one of claims 7 to 10, wherein A is a methylene group.

12. The production process according to any one of claims 7 to 11, wherein L is a diarylphosphoryloxy group.

13. The production process according to any one of claims 7 to 11, wherein L is a diphenylphosphoryloxy group.

14. The production process according to any one of claims 7 to 13, wherein R² is a carboxyl protecting group.

15. The production process according to any one of claims 7 to 14, wherein compound (4A) or the salt thereof is isolated and purified by crystallization.

16. The production process according to any one of claims 7 to 15, wherein the inert solvent in the step for preparing compound (4A) or a salt thereof is dimethylformamide, dimethylacetamide, dimethyl sulfoxide or hydrous dimethyl sulfoxide.

17. A process for the preparation of a compound or a salt thereof represented by the formula: 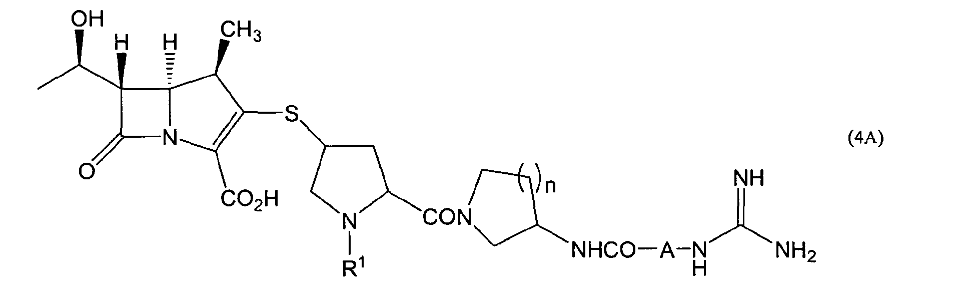 (wherein n represents 0, 1 or 2, A represents a C₁-C₃ alkylene group, and R¹ has the same meaning as defined above respectively) comprising a step for preparation of a compound or a salt thereof represented by the formula: 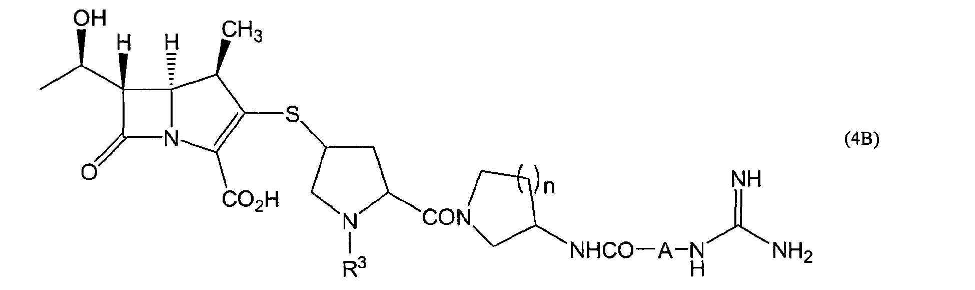 (wherein n, A have the same meanings as defined above respectively and R³ represents a hydrogen atom, a C₁-C₃ alkyl group or an amino protecting group, and the hydroxyl or carboxyl groups can independently be protected) by reacting a compound or a salt thereof represented by the formula: 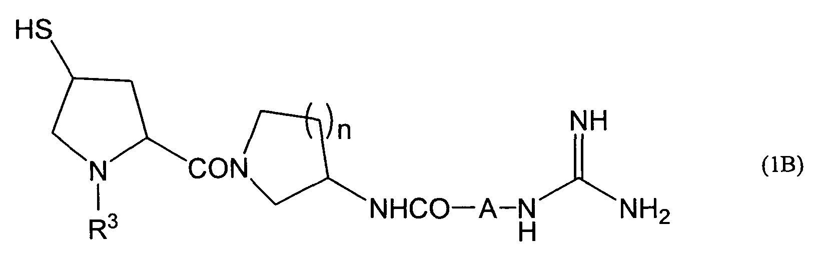 (wherein n, A and R³ have the same meanings as defined above), and a compound or a salt thereof represented by the formula: 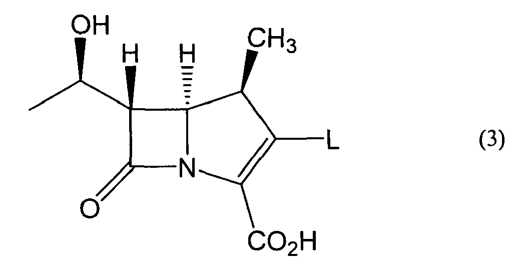 (wherein L represents a leaving group and the hydroxyl or carboxyl groups can independently be protected) in the presence of a base in an inert solvent.

18. The production process according to claim 17, wherein R¹ and R³ are each a methyl group.

19. The production process according to claim 17 or 18, wherein n is 0 or 1.

20. The production process according to claim 17 or 18, wherein n is 1.

21. The production process according to any one of claims 17 to 20, wherein A is a methylene group.

22. The production process according to any one of claims 17 to 21, wherein L is a diarylphosphoryloxy group.

23. The production process according to any one of claims 17 to 21, wherein L is a diphenylphosphoryloxy group.

24. The production process according to any one of claims 17 to 23, wherein R² is a carboxyl protecting group.

25. The production process according to any one of claims 17 to 24, wherein compound (4B) or the salt thereof is isolated and purified by crystallization.
